# FASCICULE DE BREVET EUROPEEN

(11) **EP 2 146 951 B1**
(45) Date de publication et mention de la délivrance du brevet: **01.12.2010**
(21) Numéro de dépôt: 08805701.3
(22) Date de dépôt: 24.04.2008
(51) Int. Cl.: C07C 229/52, C07D 207/06, C07D 211/14, A61K 31/235, A61P 17/00, A61Q 19/08, A61Q 5/00, A61K 8/41, A61K 31/196, A61Q 19/00

(54) **NOUVEAUX LIGANDS AGONISTES DES RECEPTEURS RARS, UTILISATION EN MEDECINE HUMAINE AINSI QU'EN COSMETIQUE**
NEUARTIGE RAR-REZEPTORAGONISTEN-LIGANDEN UND DEREN VERWENDUNG IN DER HUMANMEDIZIN UND KOSMETIK
NOVEL RAR RECEPTOR AGONIST LIGANDS AND USE THEREOF IN HUMAN MEDICINE AND COSMETICS

(30) Priorité: 11.05.2007 FR 0755019
(43) Date de publication de la demande: 27.01.2010
(73) Titulaire: Galderma Research & Development, 06410 Biot (FR)
(72) Inventeur: BIADATTI-PORTAL, Thibaud, F-06650 Opio (FR)
(74) Mandataire: Allab, Myriam
(86) Numéro de dépôt international: PCT/FR2008/050747
(87) Numéro de publication internationale: WO 2008/152260

(56) Documents cités:
- WO-A-03/101928
- FR-A- 2 840 300

## Description

L'invention se rapporte, à titre de produits industriels nouveaux et utiles, à de nouveaux composés, ligands agonistes des récepteurs RARs. Elle se rapporte également à des compositions les contenant, leurs procédés de préparation et à leur utilisation dans des compositions pharmaceutiques destinées à un usage en médecine humaine ou vétérinaire, ou bien encore dans des compositions cosmétiques et à l'utilisation non thérapeutique de ces dernières.

Les composés ayant une activité de type rétinoïde (vitamine A et ses dérivés) sont largement décrits dans la littérature, comme ayant des activités dans les processus de prolifération et différentiation cellulaire. Ces propriétés confèrent à cette classe de composés, un fort potentiel dans le traitement ou la prévention de nombreuses pathologies, et plus particulièrement en dermatologie et les cancers. La plupart des effets biologiques des rétinoïdes sont médiés par la modulation des récepteurs nucléaires de l'acide rétinoïque (RAR).

Les récepteurs RARs activent la transcription en se liant à des éléments de séquences d'ADN, appelés les éléments de réponse des RARs Element (éléments RARE) sous forme d'un hétérodimère avec les récepteurs X des rétinoïdes (appelés les RXRs).

Trois sous-types de RARs humains ont été identifiés et décrits : RAR α, RAR β, et RAR γ.

L'art antérieur contient un grand nombre de composés chimiques ligands des récepteurs de type RAR. Parmi les documents de l'art antérieur, on peut par exemple citer le brevet EP 0 816 352 B1 qui décrit des composés biaryl hétérocycliques aromatiques pour le traitement des affections dermatologiques, rhumatismales, respiratoires et ophtalmologiques, mais également pour des applications cosmétiques , le document WO 2005/056510 décrivant des molécules comprenant un cycle aromatique substitué par un radical hydroxyalkyl, les documents WO 99/10308 et WO 2006/066978 décrivant des dérivés biphényl substitués, les documents US 6 218 128 et EP 0879814 décrivant des composés bicycliques et/ou tricycliques, le document EP 0850909 décrivant des composés stilbéniques, le document WO 98/56783 décrivant des composés bi-aromatiques, les documents EP 0776885 et EP 0776881 décrivant des composés biaromatiques comportant des groupements adamantyl.

WO 03101928 divulgue aussi des composés biaromatiques inhibiteurs des récepteurs RARs.

Le sous type gamma de la famille des récepteurs RAR est largement majoritaire dans l'épiderme où il représente environ 90% du total des récepteurs (« Retinoic acid receptors and binding proteins in human skin », Elder JT, Astrom A, Pettersson U, Tavakkol A, Krust A, Kastner P, Chambon P, Voorhees JJ : J Invest Dermatol. 1992;98 (6 Suppl): 36S-41S; ou : "Retinoic acid receptor expression in human skin keratinocytes and dermal fibroblasts in vitro", Redfem CP, Todd C. J Cell Sci. 1992;102 ( Pt 1):113-21) et c'est bien l'interaction avec ce récepteur RAR gamma qui est responsable de l'efficacité des rétinoïdes sur l'épiderme (« Retinoic acid receptor gamma mediates topical retinoid efficacy and irritation in animal models», Chen S, Ostrowski J, Whiting G, Roalsvig T, Hammer L, Currier SJ, Honeyman J, Kwasniewski B, Yu KL, Sterzycki R, et al. J Invest Dermatol. 1995;104 (5): 779-83).

Les récepteurs RAR gamma sont donc la seule cible dans le traitement de pathologies au niveau de l'épiderme comme par exemple pour l'acné ou le psoriasis ou toute autre pathologie cutanée traitée par les rétinoïdes.

Or, la Demanderesse a synthétisé de nouveaux composés agonistes sélectifs RAR γ.

Ainsi, la présente invention concerne des composés de formule générale (I) suivante : 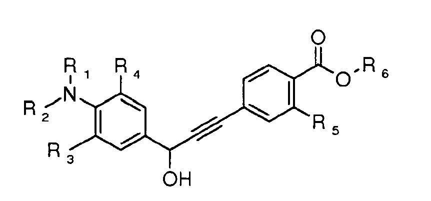 dans laquelle :
- R₁ est un atome d'hydrogène, un radical alkyle contenant de 1 à 4 atomes de carbone,
- R₂ est un radical alkyle contenant de 1 à 4 atomes de carbone,
- ou bien R₁, R₂ forment ensemble, avec l'atome d'azote N auquel ils sont rattachés, un hétérocycle de type pipéridine ou pyrrolidine,
- R₃ est un radical alkyle contenant de 1 à 4 atomes de carbone,
- R₄ est un hydrogène, un radical alkyle contenant de 1 à 4 atomes de carbone, ou un atome d'halogène,
- R₅ est un atome d'hydrogène ou un hydroxy,
- R₆ est un atome d'hydrogène, un radical alkyle contenant de 1 à 4 atomes de carbone, et les sels des composés des formule (I), notamment lorsque R₆ représente un atome d'hydrogène, ainsi que les isomères optiques desdits composés de formule (I).

Selon la présente invention, on désigne par l'expression « radical alkyle contenant de 1 à 4 atomes de carbone », une chaîne hydrocarbonée saturée, linéaire ou ramifiée comprenant de 1 à 4 atomes de carbone.

De préférence, un tel radical est choisi parmi les radicaux méthyle, éthyle, propyle, butyle, 2-méthylpropyle, i-propyle et t-butyle.

Par halogène, on entend de préférence un atome de chlore, de brome, de fluor ou d'iode.

Lorsque les composés selon l'invention se présentent sous forme de sels de la fonction acide carboxylique (R₆=H), il s'agit de préférence d'un sel d'un métal alcalin ou alcalino-terreux, ou encore d'un sel de zinc ou d'un sel d'une amine organique.

Lorsque les composés selon l'invention possèdent une fonction amine (ie quand R₁ est un atome d'hydrogène ou un radical alkyle contenant de 1 à 4 atomes de carbone, et R₂ est un radical alkyle contenant de 1 à 4 atomes de carbone) et se présentent sous forme de sels de cette amine, il s'agit de sels d'acide inorganique comme par exemple l'acide chlorhydrique, l'acide sulfurique, ou l'acide bromhydrique ou de sels d'acide organique comme par exemple l'acide acétique, l'acide triflique, l'acide tartrique, l'acide oxalique, l'acide citrique ou l'acide trifuoroacétique.

Selon la présente invention et dans un mode de réalisation avantageux, les composés de l'invention sont ceux pour lesquels l'une au moins et de préférence toutes les conditions ci-dessous sont respectées :
- R₁ est choisi parmi un atome d'hydrogène ou un radical méthyle ou éthyle
- R₂ est choisi parmi un radical méthyle, éthyle et 2-méthylpropyle ou
- R₁, R₂ forment ensemble, avec l'atome d'azote N auquel ils sont rattachés, un hétérocycle de type pipéridine ou pyrrolidine
- R₃ est choisi parmi les radicaux i-propyle et t-butyle
- R₄ est choisi parmi un atome d'hydrogène, un radical méthyle ou éthyle, un radical i-propyle et un atome de chlore
- R₅ est un atome d'hydrogène ou un hydroxy,
- R₆ est un atome d'hydrogène ou un radical alkyle contenant de 1 à 4 atomes de carbone.

Parmi les composés de formule (I) entrant dans le cadre de la présente invention, on peut notamment citer les composés suivants :
1/ - Acide 4-[(*tert*-butyl-diethylamino-phenyl)-hydroxy-prop-1-ynyl]-benzoïque.
2/- Acide 4-{[ *tert -* butyl -(ethyl-isobutyl-amino)-phenyl]-hydroxy-prop-1-ynyl}-benzoïque
3/- Acide 4-[3-(3- *tert* - butyl 4-dimethylamino-phenyl)-3-hydroxy-prop-1-ynyl]-benzoïque
4/- Acide 4-[3-(3- *tert -* butyl -4-pyrrolidin-1-yl-phenyl)-3-hydroxy-prop-1-ynyl]-benzoïque
5/- Acide 4-[3-(3- *tert* - butyl -4-piperidin-1-yl-phenyl)-3-hydroxy-prop-1-ynyl]-benzoïque
6/- Acide 4-[3-(3- *tert -* butyl -4-diethylamino-phenyl)-3-hydroxy-prop-1-ynyl]-2-hydroxy-benzoïque
7/- Acide 4-[3-(3- *tert* - butyl -5-chloro-4-dimethylamino-phenyl)-3-hydroxy-prop-1-ynyl]-benzoïque
8 - Acide 4-[3-(3- *tert -* butyl -5-chloro-4-dimethylamino-phenyl)-3-hydroxy-prop-1-ynyl]-2-hydroxy-benzoïque
9 - Acide 4-[3-(4-Dimethylamino-3,5-diisopropyl-phenyl)-3-hydroxy-prop-1-ynyl]-benzoïque
10 - Acide 4-[3-(4-Dimethylamino-3,5-diisopropyl-phenyl)-3-hydroxy-prop-1-ynyl]-2-hydroxy-benzoïque
11 - Acide 4-[3-(4-Diethylamino-3-isopropyl-phenyl)-3-hydroxy-prop-1-ynyl]-benzoïque
12 - (-) Acide 4-[3-(3- *tert -* butyl -4-diethylamino-phenyl)-3-hydroxy-prop-1-ynyl]-benzoïque
13 - (-) Acide 4-[3-(3- *tert -* butyl -4-diethylamino-phenyl)-3-hydroxy-prop-1-ynyl]-benzoïque

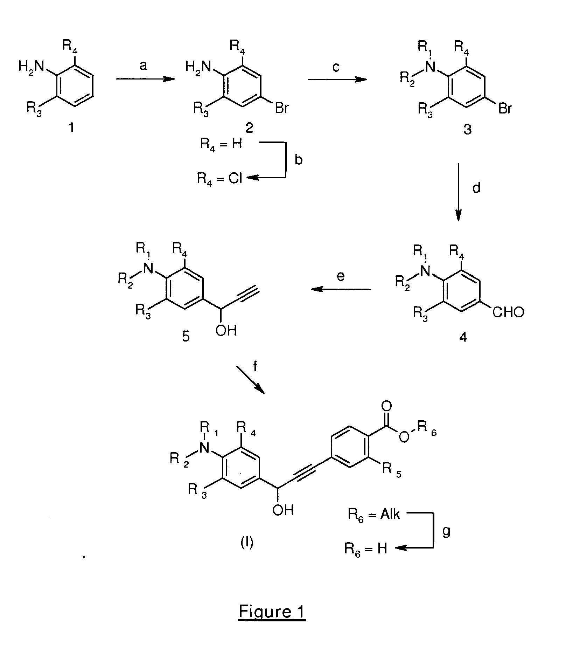

La présente invention a également pour objet les procédés de préparation des composés de formule (I), en particulier selon le schéma réactionnel donné à la figure 1.

Les intermédiaires de formule générale 2 peuvent être obtenus à partir des produits de départ 1 après une première étape de bromation en position para, selon des méthodes décrites par exemple dans Sugimoto, A. et al.; J Heterocycl Chem 1999, 36 (4), 1057-1064 ou Berthelot, J. et al.; Synth Commun 1986, 16, 1641. Dans les cas où R₄ = Cl, l'introduction du chlore peut procéder par une réaction classique d'halogénation, par exemple conformément à Silvestri, R. et al.; Org Prep Proced Int 2002, 34 (5), 507-510. Une séquence de deux réactions d'alkylation de la fonction aniline en présence de sulfate de dialkyle ou d'halogènure d'alkyle et d'une base (voir par exemple Dehmlow, E.V., Tet Lett 1985, 25, 97 ou conformément aux méthodes décrites par exemple dans « Chemistry of the amino group » par S. Patai (Wiley-Interscience , NY 1968) pages 669-682 permet d'obtenir les composés de formule 3. Dans les cas où N(R₁R₂) forme un cycle pyrrolidine ou piperidine, la formation du cycle, par exemple en présence de 1,4-dihalobutane ou 1,5-dihalopentane et d'une base ou par une méthode décrite dans « Chemistry of the amino group » par S. Patai (Wiley-Interscience , NY 1968) pages 669-682 permet d'obtenir les composés 3 correspondants. Alternativement, ces mêmes composés de formule générale 3 peuvent être générés après bromation en para, puis formation et réduction d'un groupement pyrrolidinone ou succinimide, ou piperidone (voir par exemple Ohta, S. Heterocycles 1993, 36 (4), 743 ; Hubbard, J. L.; J Heterocycl Chem 1992, 29 (4), 719 ; Akula, M. R.; Synth Commun 1998, 28 (11). 2063 ; Collins, C. J. Tetrahedron Lett 1999, 40 (19), 3673). L'introduction de la fonction aldéhyde est obtenue par réaction d'un organolithien ou organomagnésien issu de 3 et sa réaction sur un électrophile approprié comme le DMF (voir par exemple Worden, L. R. et al.; J Chem Soc C 1970, 227 ou Vuligonda, V. et al.; Bioorg Med Chem Lett 1999, 9 (5), 743-748). L'addition d'un alcynure organometallique, comme par exemple le bromure d'ethynylmagnésium, (voir par exemple Gray, M. et al.; Synlett 1992, (7), 597-598) permet la synthèse des intermédiaires de formule générale 5. Les composés de formule générale (I) peuvent alors être obtenus par couplage de type Sonogashira de ces composés 5 avec des dérivés aryles halogénés correspondants, comme par exemple l'acide 4-iodobenzoïque ou 4-iodosalicylique, ou les esters correspondants en présence de catalyseurs au Palladium. Comme précédents de littérature, on peut citer par exemple le brevet EP 661258 ou bien la publication de Sonogashira, K. et al.; Tetrahedron Lett 1975 (50), 4467. Enfin, dans le cas où R₆ = H et lorsque le couplage décrit précédemment est réalisé avec un partenaire ester au lieu du partenaire acide carboxylique, les intermédiaires avancés (I) où R₆ représente un radical alkyle contenant de 1 à 4 atomes de carbone peuvent être soumis à des réactions de saponification ou d'hydrolyse de la fonction ester en fonction acide carboxylique, par exemple en utilisant des conditions parmi celles décrites dans « Comprehensive organic transformations » par R.C. Larock, 2nd edition (J. Wiley & sons), pages 1959-1968.

Les composés selon l'invention présentent des propriétés agonistes des récepteurs de l'acide rétinoïque (RAR). Cette activité sur les récepteurs RARα, β et γ est mesurée dans un test de transactivation et quantifiée par la constance de dissociation Kdapp (apparent).

Les composés préférés de la présente invention présentent une constante de dissociation inférieure ou égale à 5000 nM, et avantageusement inférieure ou égale à 1000nM, et préférentiellement inférieure ou égale à 100 nM sur le sous type RAR γ, avec une sélectivité versus RARα et RARβ supérieure ou égale à un facteur 2 et préférentiellement à un facteur 10..

La présente invention a également pour objet les composés de formule (I) tels que décrits ci-dessus à titre de médicament.

Les composés selon l'invention conviennent particulièrement bien dans les domaines de traitement suivants :
1/ pour traiter les affections dermatologiques liées à un désordre de la kératinisation portant sur la différentiation et sur la prolifération cellulaire notamment pour traiter les acnés vulgaires, comédoniennes, polymorphes, rosacées, les acnés nodulokystiques, conglobata, les acnés séniles, les acnés secondaires telles que l'acné solaire, médicamenteuse ou professionnelle ;
2/ pour traiter d'autres types de troubles de la kératinisation, notamment les ichtyoses, les états ichtyosiformes, la maladie de Darrier, les kératodermies palmoplantaires, les leucoplasies et les états leucoplasiformes, le lichen cutané ou muqueux (buccal) ;
3/ pour traiter d'autres affections dermatologiques avec une composante immuno-allergique inflammatoire, avec ou sans trouble de la prolifération cellulaire, et notamment toutes les formes de psoriasis, qu'il soit cutané, muqueux ou unguéal, et même le rhumatisme psoriasique, ou encore l'atopie cutanée, telle que l'eczéma ou l'atopie respiratoire ou encore l'hypertrophie gingivale ;
4/ dans le traitement de désordres cutanés dus à une exposition aux rayonnements UV ainsi que pour réparer ou lutter contre le vieillissement de la peau, qu'il soit photo-induit ou chronologique ou pour réduire les pigmentations et les kératoses actiniques, ou toutes pathologies associées au vieillissement chronologique ou actinique, telle la xérose ;
5/ pour traiter toutes les proliférations dermiques ou épidermiques qu'elles soient bénignes ou malignes, qu'elles soient ou non d'origine virale telles que verrues vulgaires, les verrues planes ou l'épidermodysplasie verruciforme, les papillomatoses orales ou florides, le lymphome T, et les proliférations pouvant être induites par les ultra-violets notamment dans le cas des épithélioma baso et spinocellulaires, ainsi que toute lésion précancéreuse cutanée telles que les kératoacanthomes ;
6/ pour traiter d'autres désordres dermatologiques tels que les dermatoses immunes telles le lupus érythémateux, les maladies immunes bulleuses et les maladies du collagène, telle la sclérodermie ;
7/ dans le traitement d'affections dermatologiques ou générales à composante immunologique ;
8/ pour traiter certains troubles ophtalmologiques, notamment les cornéopathies,
9/ pour prévenir ou guérir les stigmates de l'atrophie épidermique et/ou dermique induite par les corticostéroïdes locaux ou systémiques, ou toute autre forme d'atrophie cutanée,
10/ dans le traitement de toute affection d'origine virale au niveau cutané ou général,
11/ pour lutter contre les troubles de la fonction sébacée tels que l'hyperséborrhée de l'acné ou la séborrhée simple ;
12/ pour prévenir ou traiter les troubles de la cicatrisation, ou pour prévenir ou pour réparer les vergetures, ou encore pour favoriser la cicatrisation ;
13/ dans le traitement des désordres de la pigmentation, tel l'hyperpigmentation, le mélasma, l'hypopigmentation ou le vitiligo ;
14/ dans le traitement des affections du métabolisme des lipides, tel l'obésité, l'hyperlipidémie, ou le diabète non insulino-dépendant ;
15/ dans le traitement d'affections inflammatoires telles que l'arthrite ;
16/ dans le traitement ou la prévention des états cancéreux ou précancéreux ;
17/ dans la prévention ou le traitement de l'alopécie de différentes origines, notamment l'alopécie due à la chimiothérapie ou aux rayonnements ;
18/ dans le traitement des troubles du système immunitaire, tel l'asthme, le diabète sucré de type I, la sclérose en plaque, ou autres disfonctionnements sélectifs du système immunitaire ; et
19/ dans le traitement d'affections du système cardiovasculaire telles que l'artériosclérose ou l'hypertension.

La présente invention a également pour objet une composition pharmaceutique comprenant, dans un milieu physiologiquement acceptable, au moins un composé de formule (I) tel que défini ci-dessus.

La présente invention a aussi pour objet une nouvelle composition médicamenteuse destinée notamment au traitement des affections susmentionnées, et qui est caractérisée par le fait qu'elle comprend, dans un milieu pharmaceutiquement acceptable et compatible avec le mode d'administration retenu pour cette dernière, au moins un composé de formule (I), l'un de ses isomères optiques ou un de ses sels.

L'administration de la composition selon l'invention peut être effectuée par voie orale, entérale, parentérale, topique ou oculaire. De préférence, la composition pharmaceutique est conditionnée sous une forme convenant à une application par voie topique. Par voie topique, on entend une application sur la peau ou les muqueuses.

Par voie orale, la composition peut se présenter sous forme de comprimés, de gélules, de dragées, de sirops, de suspensions, de solutions, de poudres, de granulés, d'émulsions, de suspensions de microsphères ou nanosphères ou de vésicules lipidiques ou polymériques permettant une libération contrôlée. Par voie parentérale, la composition peut se présenter sous forme de solutions ou suspensions pour perfusion ou pour injection.

Les composés selon l'invention sont généralement administrés à une dose journalière d'environ 0,01 mg/kg à 100 mg/kg de poids corporel, en une à plusieurs prises.

Les composés sont utilisés par voie systémique à une concentration généralement comprise entre 0,001% et 10% en poids, de préférence entre 0,01% et 1% en poids, par rapport au poids de la composition.

Par voie topique, la composition pharmaceutique selon l'invention est plus particulièrement destinée au traitement de la peau et des muqueuses et peut se présenter sous forme liquide, pâteuse, ou solide, et plus particulièrement sous forme d'onguents, de crèmes, de laits, de pommades, de poudres, de tampons imbibés, de syndets, de solutions, de gels, de sprays, de mousses, de suspensions, de sticks, de shampoings, ou de bases lavantes. Elle peut également se présenter sous forme de suspensions de microsphères ou nanosphères ou de vésicules lipidiques ou polymériques ou de patchs polymériques ou gélifiés permettant une libération contrôlée.

Les composés sont utilisés par voie topique à une concentration généralement comprise entre 0,001% et 10% en poids, de préférence entre 0,01% et 1% en poids, par rapport au poids total de la composition.

Les composés de formule (I) selon l'invention trouvent également une application dans le domaine cosmétique, en particulier dans l'hygiène corporelle et capillaire et notamment pour le traitement des peaux à tendance acnéique, pour la repousse des cheveux, l'anti-chute, pour lutter contre l'aspect gras de la peau ou des cheveux, dans la protection contre les aspects néfastes du soleil ou dans le traitement des peaux physiologiquement sèches, pour prévenir et/ou pour lutter contre le vieillissement photo-induit ou chronologique.

L'invention a donc également pour objet une composition cosmétique comprenant, dans un milieu physiologiquement acceptable, au moins un composé de formule (I).

L'invention a également pour objet l'utilisation non thérapeutique d'une composition cosmétique comprenant au moins un composé de formule (I) pour prévenir et/ou traiter les signes du vieillissement cutané et/ou la peau sèche.

L'invention a aussi pour objet l'utilisation non thérapeutique d'une composition cosmétique comprenant au moins un composé de formule (I) pour l'hygiène corporelle ou capillaire.

La composition cosmétique selon l'invention contenant, dans un milieu physiologiquement acceptable, au moins un composé de formule (I) ou l'un de ses isomères optiques ou géométriques ou l'un de ses sels, peut se présenter notamment sous forme d'une crème, d'un lait, d'un gel, de suspensions de microsphères ou nanosphères ou vésicules lipidiques ou polymériques, de tampons imbibés, de solutions, de sprays, de mousses, de sticks, de savons, de bases lavantes ou de shampoings.

La concentration en composé de formule (I) dans la composition cosmétique est de préférence comprise entre 0,001% et 3% en poids, par rapport au poids total de la composition.

Par un milieu physiologiquement acceptable, on entend un milieu compatible avec la peau et éventuellement avec ses phanères (cils, ongles, cheveux) et/ou les muqueuses.

Les compositions pharmaceutiques et cosmétiques telles que décrites précédemment peuvent en outre contenir des additifs inertes, ou même pharmacodynamiquement actifs pour ce qui concerne les compositions pharmaceutiques, ou des combinaisons de ces additifs, et notamment :
- des agents mouillants ;
- des agents d'amélioration de la saveur ;
- des agents conservateurs tels que les esters de l'acide parahydroxybenzoïque ;
- des agents stabilisants ;
- des agents régulateurs d'humidité ;
- des agents régulateurs de pH ;
- des agents modificateurs de pression osmotique ;
- des agents émulsionnants ;
- des filtres UV-A et UV-B ;
- des antioxydants, tels que l'α-tocophérol, le butylhydroxyanisole ou le butylhydroxytoluène, la Super Oxyde Dismutase, l'Ubiquinol ou certains chélatants de métaux ;
- des agents dépigmentants tels que l'hydroquinone, l'acide azélaïque, l'acide caféïque ou l'acide kojique ;
- des émollients ;
- des agents hydratants comme le glycérol, le PEG 400, la thiamorpholinone, et ses dérivés ou l'urée ;
- des agents antiséborrhéiques ou antiacnéiques, tels que la S-carboxyméthylcystéine, la S-benzyl-cistéamine, leurs sels ou leurs dérivés, ou le peroxyde de benzoyle ;
- des antibiotiques comme l'érythromycine et ses esters, la néomycine, la clindamycine et ses esters, les tétracyclines ;
- des agents antifongiques tels que le kétoconazole ou les polyméthylène-4,5 isothiazolidones-3 ;
- des agents favorisant la repousse des cheveux, comme le Minoxidil (2,4-diamino-6-pipéridino-pyrimidine-3-oxyde) et ses dérivés, le Diazoxide (7-chloro 3-méthyl 1,2,4-benzothiadiazine 1,1-dioxyde= et le Phénytoïne (5,4-diphényl-imidazolidine 2,4-dione) ;
- des agents anti-inflammatoires non stéroïdiens ;
- des caroténoïdes et, notamment le β-carotène ;
- des agents anti-psoriatiques tels que l'anthraline et ses dérivés ;
- des acides eicosa-5,8,11,14-tétraynoïque et eicosa-5,8,11-triynoïque, leurs esters et amides ;
- des rétinoïdes, c'est-à-dire des ligands des récepteurs RXR, naturels ou synthétiques ;
- des corticostéroïdes ou des oestrogènes ;
- des α-hydroxy acides et des α-céto acides ou leurs dérivés, tels que les acides lactique, malique, citrique, glycolique, mandélique, tartrique, glycérique, ascorbique, ainsi que leurs sels, amides ou esters, ou des β-hydroxy acides ou leurs dérivés, tels que l'acide salicylique ainsi que ses sels, amides ou esters ;
- des bloqueurs de canaux ioniques tels que les canaux potassiques ;
- ou encore, plus particulièrement pour les compositions pharmaceutiques, en association avec des médicaments connus pour interférer avec le système immunitaire (par exemple, la cyclosporine, le FK 506, les glucocorticoïdes, les anticorps monoclonaux, les cytokines ou les facteurs de croissance...).

Bien entendu, l'homme du métier veillera à choisir le ou les éventuels composés à ajouter à ces compositions de telle manière que les propriétés avantageuses attachées intrinsèquement à la présente invention ne soient pas ou substantiellement pas altérées par l'addition envisagée.

Un autre objet de l'invention se rapporte à un procédé cosmétique d'embellissement de la peau, caractérisé par le fait que l'on applique sur la peau une composition comprenant au moins un composé de formule (I) tel que défini précédemment.

L'activation des récepteurs de l'acide rétinoïque par les composés de formule (I) suivant l'invention permet d'obtenir une peau dont l'aspect de surface est embelli.

On va maintenant donner, à titre d'illustration et sans aucun caractère limitatif, plusieurs exemples d'obtention de composés actifs de formule (I) selon l'invention, des résultats d'activité biologique ainsi que diverses formulations concrètes à base de tels composés.

### Exemple 1 : Acide 4-[(tert-butyl-diethylamino-phenyl)-hydroxy-prop-1-ynyl]-benzoïque

### a' 4-Bromo-2-tert-butyl-phénylamine

25 g (168 mmol) de 2-*tert*-butylaniline sont placés dans 300 mL d'acide acétique. 450 mL d'HBr aq à 48% sont ajoutés puis le mélange est refroidi à 0°C.150 mL de DMSO sont additionnés goutte à goutte, le mélange ramené à température ambiante est agité pendant 2 heures puis il est versé sur de l'eau glacée et basifié à pH10 avec NaOH 5N. Il est extrait par de l'éther diéthylique, puis la phase organique est séchée et concentrée à sec. Le résidu est purifié par colonne (Heptane/AcOEt, 95/5). 25,2 g de 4-bromo-2-*tert*-butyl-phénylamine sont obtenus sous forme d'huile jaune (R=65%).

### b/ (4-Bromo-2-tert-butyl-phényl)-éthylamine

2,7 g (68 mmol) d'hydrure de sodium sont placés en suspension dans 250 mL de DMSO, sous un courant s'azote. 7 g (31 mmol) de 4-bromo-2-*tert*-butyl-phénylamine dilués dans 10 mL de DMSO sont additionnés au milieu réactionnel refroidi à 0°C. Après 30 minutes d'agitation à température ambiante, 5,4 mL (68 mmol) d'iodure d'éthyle sont rajoutés lentement. Le mélange de couleur jaune pâle est agité une nuit à température ambiante puis versé sur une solution saturée de chlorure d'ammonium et extrait 2 fois par de l'acétate d'éthyle. La phase organique est séchée puis concentrée sous vide. 5 g de (4-bromo-2-*tert-*butyl-phényl)-éthylamine sont obtenus sous forme d'huile jaune (R = 65%).

### c/ (4-Bromo-2-tert-butyl-phényl)-diéthylamine

5 g (20 mmol) de (4-bromo-2-*tert*-butyl-phényl)-éthylamine sont dissous sous un courant d'azote dans 200ml de DMSO. Après refroidissement à 0°C, 1,7 g (43 mmol) d'hydrure de sodium sont additionnés lentement. Après 30 minutes 3,4 mL (43 mmol) de iodoéthane sont rajoutés puis le mélange est ramené à température ambiante et agité tout un week-end. Il est ensuite versé sur une solution saturée de chlorure d'ammonium et extrait 2 fois avec de l'éther diéthylique. La phase organique est lavée à l'eau puis séchée et concentrée à sec. Le résidu est purifié par chromatographie flash (heptane/AcOEt, 95/5). 3,6 g de (4-bromo-2-*tert*-butyl-phényl)-diéthylamine sont obtenus sous forme d'huile jaune (R=63%).

### d/ 3-tert-Butyl-4-diéthylamino-benzaldéhyde

3,6 g (13 mmol) de (4-bromo-2-*tert*-butyl-phényl)-diéthylamine sont dilués dans 100ml de THF, sous un courant d'azote. Le milieu est refroidi à -78°C puis 7,8 mL (20 mmol) d'une solution de nBuLi 2.5M sont rajoutés. Après 45 minutes d'agitation, 1,5 mL (20 mmol) de DMF sont additionnés et la solution est ramenée à température ambiante. Après 15 minutes, le mélange est versé sur une solution saturée de chlorure d'ammonium puis extrait 2 fois par de l'acétate d'éthyle. La phase organique est séchée sur sulfate de sodium puis concentrée à sec. 3,1 g de 3-*tert*-butyl-4-diéthylamino-benzaldéhyde sont obtenus sous forme d'huile jaune (R=100%).

### e/ 1-(3-tert-Butyl-4-diéthylamino-phényl)-prop-2-yn-1-ol

1.5 g (6,7 mmol) de 3-*tert*-butyl-4-diéthylamino-benzaldéhyde sont dissous dans 20 mL de THF sec, sous un léger flux d'azote, à 0°C. 17 mL (8,7 mmol) d'une solution 0.5M de bromure d'éthynylmagnésium sont additionnés goutte à goutte puis le milieu est ramené à température ambiante et agité pendant 30 minutes. Le mélange est ensuite versé sur une solution saturée de chlorure d'ammonium, extrait par de l'acétate d'éthyle, séché sur sulfate de sodium puis concentré à sec. Le résidu est purifié par chromatographie flash (heptane/Acétate d'éthyle, 90/10). 780 mg de 1-(3-*tert*-butyl-4-diéthylamino-phényl)-prop-2-yn-1-ol sont obtenus sous forme d'huile incolore (R=47%).

### f/ Acide 4-[3-(3-tert-butyl-4-diéthylamino-phényl)-3-hydroxy-prop-1-ynyl]-benzoïque

390 mg de 1-(3-*tert*-butyl-4-diéthylamino-phényl)-prop-2-yn-1-ol (1,6 mmol) et 324 mg (12 mmol) d'acide 4-iodobenzoïque sont dilués dans 20 mL de triéthylamine et 13 mL de DMF sous un léger flux d'azote avec 12 mg (0,06 mmol) d'iodure de cuivre. Après 10 minutes pendant lesquelles de l'azote bulle dans le milieu, 23 mg (0,03 mmol) de chlorure de bis (triphénylphosphine) palladium(II) sont ajoutés. Après 7 heures d'agitation à température ambiante, le mélange est versé sur une solution saturée de chlorure d'ammonium puis la phase aqueuse est amenée à pH5 avec HCl 1N. Après extraction par de l'acétate d'éthyle, la phase organique est séchée sur sulfate de sodium puis concentrée à sec et le résidu est purifié par chromatographie sur silice (heptane/Acétate d'éthyle60/40 puis heptane/acétate d'éthyle: 80/20).

L'acide 4-[3-(3-*tert*-butyl-4-diéthylamino-phényl)-3-hydroxy-prop-1-ynyl]-benzoïque est obtenu sous forme de solide beige (m = 220 mg, R = 45%, pf = 140°C)

### RMN ¹H (CDCl₃, 400MHz) :

1.00 (t, J=7.2Hz, 6H); 1.43 (s, 9H) ; 2.50 (m, 4H) ; 5.58 (d, J=6Hz, 1H); 6.13 (d, J=6.4Hz, 1H); 7.29 (d, J=8Hz, 1H); 7.37 (d, J=2Hz, 1H); 7.55 (m, 3H) ; 7.93 (d, J=8.4Hz, 2H); 13.2 (m, 1H).

### Exemple 2 : Acide 4-{[tert-butyl-(ethyl-isobutyl-amino)-phenyl]-hydroxy-prop-1-ynyl}-benzoïque

### a/ (4-Bromo-2-tert-butyl-phényl)-éthyl-isobutyl-amine

De manière analogue à l'exemple 1 c, par réaction de 4 g (16 mmol) de (4-bromo-2-*tert-*butyl-phényl)-éthylamine (exemple 1 b) avec 1.4 g (34 mmol) d'hydrure de sodium 60% avec 3,7 mL (34 mmol) de 1-bromo-2-méthyl propane. 1,5 g de (4-bromo-2-*tert*-butyl-phényl)-éthyl-isobutyl-amine sont obtenus sous forme d'huile (R = 30%).

### b/ 3-tert-Butyl-4-(éthyl-isobutyl-amino)-benzaldéhyde

De manière analogue à l'exemple 1 d, par réaction de 2 g (5 mmol) de (4-bromo-2-*tert-*butyl-phényl)-éthyl-isobutyl-amine avec 3 mL (7 mmol) d'une solution 2.5 M de n-butyllithium et 0,6 mL (7 mmol) de DMF. 800 mg de 3-*tert*-butyl-4-(éthyl-isobutyl-amino)-benzaldéhyde sont obtenus sous forme d'huile jaune (R = 62%).

### c/ 1-[3-tert-Butyl-4-(éthyl-isobutyl-amino)-phényl]-prop-2-yn-1-ol

De manière analogue à l'exemple 1 e, par réaction de 800 mg (3 mmol) de 3-*tert*-butyl-4-(éthyl-isobutyl-amino)-benzaldéhyde avec 8 mL (4 mmol) d'une solution 0.5M de bromure d'éthynylmagnésium. 700 mg de 1-[3-*tert*-butyl-4-(éthyl-isobutyl-amino)-phényl]-prop-2-yn-1-ol sont obtenus sous forme d'huile incolore (R = 81%).

### d/ Acide 4-{[tert-butyl-(ethyl-isobutyl-amino)-phenyl]-hydroxy-prop-1-ynyl}-benzoïque

De manière analogue à l'exemple 1 e, par réaction de 300 mg (1 mmol) de 1-[3-*tert*-butyl-4-(éthyl-isobutyl-amino)-phényl]-prop-2-yn-1-ol avec 216 mg (0,87 mmol) d' acide 4-iodobenzoïque, 12 mg (0,04 mmol) d'iodure de cuivre et 15 mg (0,02 mmol) de chlorure de bis (triphénylphosphine) palladium. 190 mg d'acide 4-{[*tert*-butyl-(ethyl-isobutyl-amino)-phenyl]-hydroxy-prop-1-ynyl}-benzoïque sont obtenus sous forme de solide beige (Pf = 117°C, R = 53%).
RMN¹H (CDCl₃, 400MHz) :
0.86 (m, 9H), 1.44(s, 9H), 1.99 (m, 2H) ; 2.50 (m, 1H), 2.80 (m, 3H), 5.59 (d, J=6Hz, 1H), 6.13 (d, J=6Hz, 1H), 7.35 (m, 2H), 7.55 (d, J=7.6Hz, 3H), 7.93 (d, J=8Hz, 2H), 13.1 (m, 1H).

### Exemple 3 : Acide 4-[3-(3-tert-butyl-4-dimethylamino-phenyl)-3-hydroxy-prop-1-ynyl]-benzoïque

### a/ (4-Bromo-2-tert-butyl-phényl)-méthylamine

De manière analogue à l'exemple 1 b, par réaction de 12 g (53 mmol) de 4-bromo-2-*tert-*butyl-phénylamine avec 4.7 g (117 mmol) d'hydrure de sodium et 7,2 mL (117 mmol) d'iodure de méthyle. 7,4 g de (4-bromo-2-*tert*-butyl-phényl)-méthyl-amine sont obtenus sous forme d'huile jaune (R = 58%).

### b/ (4-Bromo-2-tert-butyl-phényl)-diméthylamine

De manière analogue à l'exemple 1 c, par réaction de 7,4 g (31 mmol) de (4-bromo-2-*tert-*butyl-phényl)-méthylamine avec 2,7 g (67 mmol) d'hydrure de sodium 60% avec 7,2 mL (67 mmol) de iodomethane. 4,1 g de (4-bromo-2-*tert*-butyl-phényl)-diméthylamine sont obtenus sous forme d'huile (R = 52%).

### c/ 3-tert-Butyl-4-dimethylamino-benzaldéhyde

De manière analogue à l'exemple 1 d, par réaction de 4,1 g (16 mmol) de (4-bromo-2-*tert-*butyl-phényl)-diméthylamine avec 9,6 mL (24 mmol) d'une solution 2.5 M de n-butyllithium et 1,9 mL (24 mmol) de DMF. 2 g de 3-*tert*-butyl-4-dimethylamino-benzaldéhyde sont obtenus sous forme d'huile jaune (R =100%).

### d/ 1-(3-tert-Butyl-4-diméthylamino-phényl)-prop-2-yn-1-ol

De manière analogue à l'exemple 1 e, par réaction de 1 g (4,9 mmol) de 3-*tert*-butyl-4-dimethylamino-benzaldéhyde avec 12 mL (6 mmol) d'une solution 0.5M de bromure d'éthynylmagnésium. 900 mg de 1-(3-*tert*-butyl-4-diméthylamino-phényl)-prop-2-yn-1-ol sont obtenus sous forme d'huile incolore (R = 79%).

### e/ Acide 4-[3-(3-tert-butyl-4-dimethylamino-phenyl)-3-hydroxy-prop-1-ynyl]-benzoïque

De manière analogue à l'exemple 1 e, par réaction de 900 mg (3,8 mmol) de 1-(3-*tert*-butyl-4-diméthylamino-phényl)-prop-2-yn-1-ol avec 805 mg (3,2 mmol) d' acide 4-iodobenzoïque, 30 mg (0,16 mmol) d'iodure de cuivre et 56 mg (0,08 mmol) de chlorure de bis (triphénylphosphine) palladium. 700 mg d'acide 4-[3-(3-*tert*-butyl-4-dimethylamino-phenyl)-3-hydroxy-prop-1-ynyl]-benzoïque sont obtenus sous forme de solide beige (Pf = 139°C, R = 64%).

RMN¹H (CDCl₃, 400MHz) : 1.48 (s, 9H), 2.63(s, 6H), 5.69 (s, 1H), 7.42 (d, J=8Hz, 1H), 7.50 (m, 1H), 7.58 (s, 1H), 7.60(m, 2H), 8.08 (d, J=8Hz, 2H).

### Exemple 4 : Acide 4-[3-(3-tert-butyl-4-pyrrolidin-1-yl-phenyl)-3-hydroxy-prop-1-ynyl]-benzoïque

### a/ 1-(4-Bromo-2-tert-butyl-phényl)-pyrrolidine

De manière analogue à l'exemple 1 b, par réaction de 10 g (44 mmol) de 4-bromo-2-*tert-*butyl-phénylamine avec 3,8 g (96 mmol) d'hydrure de sodium 60% et avec 3,4 mL (96 mmol) de 1,4-dibromobutane. 7,6 g de 1-(4-bromo-2-*tert*-butyl-phényl)-pyrrolidine sont obtenus sous forme d'huile (R = 61%).

### b/ 3-tert-Butyl-4-pyrrolidin-1-yl-benzaldéhyde

De manière analogue à l'exemple 1 d, par réaction de 7,6 g (27 mmol) de 1-(4-bromo-2-*tert-*butyl-phényl)-pyrrolidine avec 16,2 mL (40 mmol) d'une solution 2.5 M de n-butyllithium et 3,1 mL (40 mmol) de DMF. 5 g de 3-*tert*-butyl-4-pyrrolidin-1-yl-benzaldéhyde sont obtenus sous forme d'huile jaune (R =80%).

### c/ 1-(3-tert-Butyl-4-pyrrolidin-1-yl-phényl)-prop-2-yn-1-ol

De manière analogue à l'exemple 1 e, par réaction de 1 g (4 mmol) de 3-*tert*-butyl-4-pyrrolidin-1-yl-benzaldéhyde avec 11 mL (5,5 mmol) d'une solution 0.5M de bromure d'éthynylmagnésium. 640 mg de 1-(3-*tert*-butyl-4-pyrrolidin-1-yl-phényl)-prop-2-yn-1-ol sont obtenus sous forme d'huile incolore (R = 62%).

### d/ Acide 4-[3-(3-tert-butyl-4-pyrrolidin-1-yl-phenyl)-3-hydroxy-prop-1-ynyl]-benzoïque

De manière analogue à l'exemple 1 e, par réaction de 640 mg (2,5 mmol) de 1-(3-*tert*-butyl-4-pyrrolidin-1-yl -hényl)-prop-2-yn-1-ol avec 520 mg (2,1 mmol) d' acide 4-iodobenzoïque, 20 mg (0,1 mmol) d'iodure de cuivre et 37 mg (0,05 mmol) de chlorure de bis (triphénylphosphine) palladium. 360 mg d'acide 4-[3-(3-*tert*-butyl-4-pyrrolidin-1-yl-phenyl)-3-hydroxy-prop-1-ynyl]-benzoïque sont obtenus sous forme de solide beige (Pf = 150°C, R = 46%).

RMN¹H (CDCl₃, 400MHz) : 1.39 (s, 9H), 1.87 (m, 4H), 2.91 (m, 4H), 4.10 (s, 1H), 5.60 (s, 1H), 7.34 (d, J=8Hz, 1H), 7.45 (m, 3H), 7.55 (d, J=2Hz, 1H), 7.94 (s, 1H), 7.96 (s, 1H).

### Exemple 5 : Acide 4-[3-(3-tert-butyl-4-piperidin-1-yl-phenyl)-3-hydroxy-prop-1-ynyl]-benzoïque

### a/ 1-(4-Bromo-2-tert-butyl-phényl)-piperidine

De manière analogue à l'exemple 1 b, par réaction de 10 g (44 mmol) de 4-bromo-2-*tert-*butyl-phénylamine avec 3,8 g (96 mmol) d'hydrure de sodium 60% et avec 3,4 mL (96 mmol) de 1,4-dibromobutane. 9,9 g de 1-(4-bromo-2-*tert*-butyl-phényl)-piperidine sont obtenus sous forme d'huile (R = 76%).

### b/ 3-tert-Butyl-4-piperidin-1-yl-benzaldéhyde

De manière analogue à l'exemple 1 d, par réaction de 9,9 g (34 mmol) de 1-(4-bromo-2-*tert-*butyl-phényl)-piperidine avec 20 mL (50 mmol) d'une solution 2.5 M de n-butyllithium et 3,9 mL (50 mmol) de DMF. 8,8 g de 3-*tert*-butyl-4-piperidin-1-yl-benzaldéhyde sont obtenus sous forme d'huile jaune (R =100%).

### c/ 1-(3-tert-Butyl-4-piperidin-1-yl-phényl)-prop-2-yn-1-ol

De manière analogue à l'exemple 1 e, par réaction de 1 g (4 mmol) de 3-*tert*-butyl-4-pipcridin-1-yl-benzaldéhyde avec 12 mL (6 mmol) d'une solution 0.5M de bromure d'éthynylmagnésium. 890 mg de 1-(3-*tert*-butyl-4-piperidin-1-yl-phényl)-prop-2-yn-1-ol sont obtenus sous forme d'huile incolore (R = 82%).

### d/ Acide 4-[3-(3-tert-butyl-4-piperidin-1-yl-phenyl)-3-hydroxy-prop-1-ynyl]-benzoïque

De manière analogue à l'exemple 1 e, par réaction de 1,18 g (4,4 mmol) de 1-(3-*tert*-butyl-4-piperidin-1-yl -hényl)-prop-2-yn-1-ol avec 890 mg (3,6 mmol) d' acide 4-iodobenzoïque, 35 mg (0,18 mmol) d'iodure de cuivre et 63 mg (0,09 mmol) de chlorure de bis (triphénylphosphine) palladium. 650 mg d'acide 4-[3-(3-*tert*-butyl-4-piperidin-1-yl-phenyl)-3-hydroxy-prop-1-ynyl]-benzoïque sont obtenus sous forme de solide beige (Pf = 112°C, R = 46%).

RMN¹H (CDCl₃, 400MHz) : 1.41 (s, 9H), 1.57 (m, 4H), 1.65 (m, 1H), 2.68 (m, 5H), 5.57 (d, J=4Hz, 1H), 6.13 (d, J=8Hz, 1H), 7.38 (s, 2H), 7.49 (s, 1H), 7.54 (d, J=8Hz, 2H), 7.92 (d, J=8Hz, 2H).

### Exemple 6 : Acide 4-[3-(3-tert-butyl-4-diethylamino-phenyl)-3-hydroxy-prop-1-ynyl]-2-hydroxy-benzoïque

### a 4-[3-(3-tert-butyl-4-diethylamino-phenyl)-3-hydroxy-prop-1-ynyl]-2-hydroxy-benzoate de méthyle

De manière analogue à l'exemple 1 e, par réaction de 3,7 g (15 mmol) de 1-(3-*tert*-butyl-4-diéthylamino-phényl)-prop-2-yn-1-ol avec 3,3 g (12 mmol) de 4-iodosalicylate de méthyle, 114 mg (0.6 mmol) d'iodure de cuivre et 210 mg (0,3 mmol) de chlorure de bis (triphénylphosphine) palladium. 4 g de 4-[3-(3-*tert*-butyl-4-diethylamino-phenyl)-3-hydroxy-prop-1-ynyl]-2-hydroxy-benzoate de méthyle sont obtenus sous forme d'huile jaune (R = 66%).

### b/ Acide 4-[3-(3-tert-butyl-4-diethylamino-phenyl)-3-hydroxy-prop-1-ynyl]-2-hydroxy-benzoïque

1,7 g (4,3 mmol) de 4-[3-(3-*tert*-butyl-4-diethylamino-phenyl)-3-hydroxy-prop-1-ynyl]-2-hydroxy-benzoate de méthyle sont dissous dans 50 mL de THF avec 1 mL de méthanol et 2 mL de NaOH aq 1N. La solution est agitée à température ambiante 2 heures puis 2 jours à reflux. Le milieu réactionnel est versé sur une solution saturée de chlorure d'ammonium, la phase aqueuse est amenée à pH acide avec HCl 1N puis extraite deux fois par de l'acétate d'éthyle. La phase organique est séchée sur sulfate de magnésium puis concentrée à sec. Le résidu est purifié par chromatographie (heptane/acétate d'éthyle : 50/50). 760 mg d'acide 4-[3-(3-*tert*-butyl-4-diethylamino-phenyl)-3-hydroxy-prop-1-ynyl]-2-hydroxy-benzoïque sont obtenus sous forme d'un solide blanchâtre (Pf = 245°C, R = 45%).

RMN ¹H (DMSO, 400MHz) : 1.01 (t, J=8Hz, 6H), 1.44 (s, 9H), 2.85 (m, 4H), 5.58 (s, 1H), 6.10 (s, 1H), 6.97 (d, J=6.4Hz, 2H), 7.29 (d, J=8Hz, 1H), 7.37 (d, J=8Hz, 1H), 7.55 (s, 1H), 7.77 (d, J=8.4Hz, 1H).

### Exemple 7 : Acide 4-[3-(3-tert-butyl-5-chloro-4-dimethylamino-phenyl)-3-hydroxy-prop-1-ynyl]-benzoïque

### a/ 4-bromo-2-tert-butyl-6-chloro-phenylamine.

6.4 g (48 mmol) de N-Chlorosuccinimide sont ajoutés à une solution de 10 g (44 mmol) de 4-bromo-2-*tert*-butyl-phenylamine (exemple 1 a) dans 150 mL de DMF. Le milieu est chauffé à 70°C pendant 2 heures. Il est alors versé dans une solution aqueuse saturée en chlorure de sodium et extrait avec de l'acétate d'éthyle. Les phases organiques sont rassemblées et lavées à l'eau puis séchées sur sulfate de sodium. Le résidu est chromatographié sur gel de silice (Heptane/Acétate d'éthyle 90/10). 11.4 g de 4-bromo-2-*tert*-butyl-6-chloro-phenylamine sont obtenus sous la forme d'une huile orange (R = 99%).

### b/ (4-bromo-2-tert-butyl-6-chloro-phenyl)-methyl-amine

De manière analogue à l'exemple 1 b, par réaction de 3 g (11,4 mmol) de 4-bromo-2-*tert-*butyl-6-chloro-phenylamine avec 1 g (25 mmol) d'hydrure de sodium et 1.6 mL (25.7 mmol) d'iodure de méthyle. 2,54 g de (4-bromo-2-*tert*-butyl-6-chloro-phenyl)-methyl-amine sont obtenus sous la forme d'une huile orange (R = 80%).

### c/ (4-bromo-2-tert-butyl-6-chloro-phenyl)-dimethyl-amine

De manière analogue à l'exemple 1 c, par réaction de 2,54 g (9,2 mmol) de (4-bromo-2-*tert-*butyl-6-chloro-phenyl)-methyl-amine avec 850 mg (21,3 mmol) d'hydrure de sodium et 1,3 mL (21 mmol) d'iodure de méthyle. 2 g de (4-bromo-2-*tert*-butyl-6-chloro-phenyl)-dimethyl-amine sont obtenus sous forme d'huile jaune (R = 75%).

### d/ 3-tert-butyl-5-chloro-4-dimethylamino-benzaldehyde .

De manière analogue à l'exemple 1 d par réaction de 2,25 mL (5,6 mmol) de n-butyllithium 2.5M/Hexane avec 1,5 g (5,2 mmol) de (4-bromo-2-*tert*-butyl-6-chloro-phenyl)-dimethyl-amine et 450 µl (5,8 mmol) de diméthylformamide. 1.23 g de 3-*tert*-butyl-5-chloro-4-dimethylamino-benzaldehyde sont obtenus sous forme d'huile jaune (R = 99%).

### c/ 1-(3-tert-butyl-5-chloro-4-dimethylamino-phenyl)-prop-2-yn-1-ol

De manière analogue à l'exemple 1 e par réaction de 13 mL (6,5 mmol) de bromure d'éthynylmagnesium *0.5 M*/*THF* avec 1.2 g (5 mmol) de 3-*tert*-butyl-5-chloro-4-dimethylamino-benzaldehyde. 650 mg de 1-(3-*tert*-butyl-5-chloro-4-dimethylamino-phenyl)-prop-2-yn-1-ol sont obtenus sous la forme d'un solide jaune (R = 49%).

### f/ Acide 4-[3-(3-tert-butyl-5-chloro-4-dimethylamino-phenyl)-3-hydroxy-prop-1-ynyl]-benzoïque.

De manière analogue à l'exemple 1 f par réaction de 200 mg (0,8 mmol) d'acide 4-iodobenzoïque avec 300 mg (1,1 mmol) de 1-(3-*tert*-butyl-5-chloro-4-dimethylamino-phenyl)-prop-2-yn-1-ol en présence de 8 mg (0,04 mmol) d'iodure de cuivre et 16 mg (0,02 mmol) de chlorure de bis (triphénylphosphine) palladium. 270 mg d'acide 4-[3-(3-*tert*-butyl-5-chloro-4-dimethylamino-phenyl)-3-hydroxy-prop-1-ynyl]benzoïque sont obtenus sous la forme d'un solide blanchâtre (Pf= 120°C, R = 87%).

RMN ¹H (CDCl₃, 400 Mz): 1.45 (s, 9H); 2.84 (s, 6H); 5.65 (s, 1H); 7.49- 7.50 (d, 1H, *J*= 2 Hz); 7.53- 7.54 (d, 1H, *J*= 2 Hz); 7.58- 7.60 (d, 2H, *J*= 6.85 Hz); 8.08- 8.1 (d, 2H, J= 6.85 Hz).

### Exemple 8 : Acide 4-[3-(3-tert-butyl-5-chloro-4-dimethylamino-phenyl)-3-hydroxy-prop-1-ynyl]-2-hydroxy-benzoïque

### a/ Acide 4-[3-(3-tert-butyl-5-chloro-4-dimethylamino-phenyl)-3-hydroxy-prop-1-ynyl]-2-hydroxy-benzoïque.

De manière analogue à l'exemple 1 f par réaction de 220 mg (0,83 mmol) d'acide 2-hydroxy-4-iodo-benzoïque avec 320 mg (1,2 mmol) de 1-(3-*tert*-butyl-5-chloro-4-dimethylamino-phenyl)-prop-2-yn-1-ol en présence de 9 mg (0,05 mmol) d'iodure de cuivre et 17 mg (0,025 mmol) de chlorure de bis (triphénylphosphine) palladium. 140 mg d'acide 4-[3-(3-*tert*-butyl-5-chloro-4-dimethylamino-phenyl)-3-hydroxy-prop-1-ynyl]-2-hydroxy-benzoïque sont obtenus sous la forme d'un solide jaunâtre (Pf= 175°C, R = 42%).

RMN ¹H (CDCl₃, 400 Mz): 1.39 (s, 9H); 2.77 (s, 6H); 5.55 (s, 1H); 6.90-6.93 (dd, 1H, *J*= 1.44 Hz, J'= 6.72 Hz); 7.0 (d, 1H, *J*= 1.32 Hz); 7.42-7.43 (d, 1H, *J*= 2.08 Hz); 7.47-7.48 (d, 1H, *J*= 2.04 Hz); 7.76-7.78 (d, 1H, *J*= 8.12 Hz).

### Exemple 9 : Acide 4-[3-(4-dimethlamino-3,5-diisopropyl-phenyl)-3-hydroxy-prop-1-ynyl]-benzoïque

### a/ 4-bromo-2,6-diisopropyl-phenylamine.

40,8 g (85 mmol) de tribromure de tetra-n-butyl ammonium sont ajoutés à 0°C et par portions à une solution de 15 g (85 mmol) de 2,6-diisopropyl-phenylamine dans 200 mL de tetrahydrofuranne. Le milieu est agité pendant 2 h. Il est alors versé dans une solution aqueuse saturée en thiosulfate de sodium et extrait avec de l'acétate d'éthyle. Les phases organiques sont rassemblées et lavées avec de l'eau. Elles sont séchées sur sulfate de sodium. Le résidu est purifié par chromatographie sur gel de silice (Heptane/Acétate d'éthyle 80/20). 20.6 g de 4-bromo-2,6-diisopropyl-phenylamine sont obtenus sous la forme d'une huile jaune (R = 95%).

### b/ (4-bromo-2,6-diisopropyl-phenyl)-dimethyl-amine

Une solution de 12 g (47 mmol) de 4-bromo-2,6-diisopropyl-phenylamine dans 100 mL de DMSO est ajoutée sur 4.1 g (100 mmol) d'hydrure de sodium dans 20 mL DMSO. Le milieu est agité pendant 1 h puis 6.4 mL (100 mmol) d'iodure de méthyle sont additionnés. Le milieu est chauffé à 45°C pendant 3 heures puis versé dans une solution aqueuse saturée en chlorure d'ammonium et extrait avec de l'acétate d'éthyle. Les phases organiques sont rassemblées et lavées à l'eau. Elles sont séchées sur sulfate de sodium. Le résidu est purifié par chromatographie sur gel de silice (Heptane/Acétate d'éthyle 90/10). 8.7 g de (4-bromo-2,6-diisopropyl-phenyl)-dimethylamine sont obtenus sous forme d'un solide blanc (R = 65%).

### c/ 4-dimethylamino-3,5-diisopropyl-benzaldehyde.

De manière analogue à l'exemple 1 d par réaction de 5,5 mL (13,8 mmol) de n-butyllithium 2.5M/Hexane avec 3,3 g (11,6 mmol) de 4-bromo-2,6-diisopropyl-phenyl)-dimethylamine et 1,1 mL (14 mmol) de diméthylformamide. 2,6 g de 4-dimethylamino-3,5-diisopropyl-benzaldehyde sont obtenus sous forme d'huile jaune (R = 96%).

### d/ 1-(4-dimethylamino-3,5-diisopropyl-phenyl)-prop-2-yn-1-ol

De manière analogue à l'exemple 1 e par réaction de 28 mL (14 mmol) de bromure d'éthynylmagnesium *0.5 M*/*THF* avec 1.2 g (5 mmol) de 4-dimethylamino-3,5-diisopropyl-benzaldehyde. 2.5 g de 1-(4-dimethylamino-3,5-diisopropyl-phenyl)-prop-2-yn-1-ol sont obtenus sous forme d'un solide blanchâtre (R = 90%).

### e/ Acide 4-[3-(4-dimethylamino-3,5-diisopropyl-phenyl)-3-hydroxy-prop-1-ynyl]benzoïque

De manière analogue à l'exemple 1 f par réaction de 335 mg (1,35 mmol) d'acide 4-iodobenzoïque avec 500 mg (1,9 mmol) de 1-(4-dimethylamino-3,5-diisopropyl-phenyl)-prop-2-yn-1-ol en présence de 15 mg (0.08 mmol) d'iodure de cuivre et 27 mg (0.04 mmol) de chlorure de bis (triphénylphosphine) palladium. 190 mg d'acide-4-[3-(3-*tert*-butyl-5-chloro-4-dimethylamino-phenyl)-3-hydroxy-prop-1-ynyl]benzoïque sont obtenus sous la forme d'un solide blanchâtre (Pf= 198°C, R = 37%).

RMN ¹H (CDCl₃, 400 Mz): 1.25 (s, 6H); 1.27 (s, 6H); 2.85 (s, 3H); 2.87 (s, 3H); 3.37- 3.41 (c, 2H); 5.68 (s, 1H); 7.34 (s, 2H); 7.58- 7.60 (d, 2H, *J*= 8.4 Hz); 8.07- 8.1 (d, 2 H, *J*= 8.4 Hz)

### Exemple 10 : Acide 4-[3-(4-dimethylamino-3,5-diisopropyl-phenyl)-3-hydroxy-prop-1-ynyl]-2-hydroxy-benzoïque

### a/ Acide 4-[3-(4-dimethylamino-3,5-diisopropyl-phenyl)-3-hydroxy-prop-1-ynyl]-2-hydroxy-benzoïque

De manière analogue à l'exemple 1 f par réaction de 360 mg (1,35 mmol) d'acide 2-hydroxy-4-iodo-benzoïque avec 500 mg (1,9 mmol) de 1-(4-dimethylamino-3,5-diisopropyl-phenyl)-prop-2-yn-1-ol en présence de 15 mg (0,08 mmol) d'iodure de cuivre et 27 mg (0,04 mmol) de chlorure de bis (triphénylphosphine) palladium. 120 mg d'acide 4-[3-(4-dimethylamino-3,5-diisopropyl-phenyl)-3-hydroxy-prop-1-ynyl]-2-hydroxy-benzoïque sont obtenus sous la forme d'un solide jaunâtre (Pf: dec>250°C, R = 22%).

RMN ¹H (CDCl₃, 400 Mz): 1.19 (s, 6H); 1.21 (s, 6H); 2.79 (s, 3H); 2.81 (s, 3H); 3.31- 3.34 (c, 2H); 5.59 (s, 1H); 6.91- 6.93 (dd, 1 H, *J*= 1.44 Hz, *J*'= 6.76 Hz); 7.01 (d, 1 H, *J*= 1.24 Hz); 7.28 (s, 2H); 7.77- 7.79 (d, 1H, *J*= 8.16 Hz); 11.25 (s, 1 H).

### Exemple 11 : Acide 4-[3-(4-diethylamino-3-isopropyl-phenyl)-3-hydroxy-prop-1-ynyl]-benzoïque

### a/ 4-bromo-2-isopropyl-phenylamine.

De manière analogue à l'exemple 9 a, par réaction de 89 g (185 mmol) de tribromure de tetra-n-butyl ammonium avec 25 g (185 mmol) de 2-isopropylaniline. 22 g de 4-bromo-2-isopropyl-phenylamine sont obtenus (R = 57%).

### b/ (4-bromo-2-isopropyl-phenyl)-ethylamine

De manière analogue à l'exemple 9 b, par réaction de 6,9 g (32 mmol) de 4-bromo-2-isopropyl-phenylamine avec 2,8 g (70 mmol) d'hydrure de sodium et 5.7 mL (71 mmol) d'iodure d'éthyle.7,4 g de (4-bromo-2-isopropyl-phenyl)-ethylamine brut sont obtenus.

### c/ (4-bromo-2-isopropyl-phenyl)-diethylamine

De manière analogue à l'exemple 9 c, par réaction de 7,4 g de (4-bromo-2-isopropyl-phenyl)-ethylamine avec 2,8 g (70 mmol) d'hydrure de sodium et 5.7 mL (71 mmol) d'iodure d'éthyle. 4,8 g de (4-bromo-2-isopropyl-phenyl)-diéthylamine sont obtenus (R = 56%).

### d/ 4-diethylamino-3-isopropyl-benzaldehyde .

De manière analogue à l'exemple 1 d par réaction de 1,8 mL (4,4 mmol) de n-butyllithium 2.5M/Hexane avec 1 g (3,7 mmol) de (4-bromo-2-isopropyl-phenyl)-diethyl-amine et 0,35 mL (4,5 mmol) de diméthylformamide. 790 mg de 4-diethylamino-3-isopropyl-benzaldehyde sont obtenus sous forme d'huile jaune (R = 97%).

### e/ 1-(4-diethylamino-3-isopropyl-phenyl)-prop-2-yn-1-ol

De manière analogue à l'exemple 1 e par réaction de 9,3 mL (4,7 mmol) de bromure d'éthynylmagnesium 0.5 M/THF avec 780 mg (3,5 mmol) de 4-diethylamino-3-isopropyl-benzaldehyde. 720 mg de 1-(4-diethylamino-3-isopropyl-phenyl)-prop-2-yn-1-ol sont obtenus sous forme d'une huile jaune (R = 83%).

### f/ Acide 4-[3-(4-diethylamino-3-isopropyl-phenyl)-3-hydroxy-prop-1-ynyl]-benzoïque

De manière analogue à l'exemple 1 f par réaction de 248 mg (1 mmol) d'acide 4-iodobenzoïque avec 350 mg (1,4 mmol) de 1-(4-diethylamino-3-isopropyl-phenyl)-prop-2-yn-1-ol en présence de 11 mg (0,06 mmol) d'iodure de cuivre et 20 mg (0,03 mmol) de chlorure de bis (triphénylphosphine) palladium. 240 mg d'acide 4-[3-(4-diethylamino-3-isopropyl-phenyl)-3-hydroxy-prop-1-ynyl]benzoïque sont obtenus sous la forme d'un solide blanchâtre (Pf= 145°C, R = 46%).

RMN ¹H (CDCl₃, 400 Mz): 0.99 (s, 3H); 1.01 (s, 3H); 1.21 (s, 3H); 1.23 (s, 3H); 2.98- 2.99 (c, 4H); 3.67- 3.72 (c, 1 H); 5.70 (s, 1H); 7.17- 7.19 (d, 1 H, *J*= 8.22 Hz); 7.41- 7.43 (dd, 1 H, *J*= 1.8 Hz, *J*'= 6.26 Hz); 7.52 (d, 1 H, *J*= 1.63 Hz); 7.58- 7.6 (d, 2 H, *J*= 8.22 Hz); 8.07-8.10 (d, 2 H, *J*= 8.22 Hz).

### Exemple 12 : Test de transactivation

L'activation des récepteurs par un agoniste (activateur) dans des cellules HeLa conduit à l'expression d'un gène reporter, la luciférase, qui, en présence d'un substrat génère de la lumière. On peut donc mesurer l'activation des récepteurs en quantifiant la luminescence produite après incubation des cellules en présence d'un agoniste de référence. Les produits inhibiteurs vont déplacer l'agoniste de son site empêchant ainsi l'activation du récepteur. La mesure de l'activité se fait par la quantification de la baisse de la lumière produite. Cette mesure permet de déterminer l'activité inhibitrice des composés selon l'invention.

Dans cette étude, nous déterminons une constante qui représente l'affinité de la molécule pour le récepteur. Celle valeur pouvant fluctuer selon l'activité basale et l'expression du récepteur, on la dénomme Kd apparent (KdApp).

Pour déterminer cette constante, des « courbes croisées » du produit à tester contre un agoniste de référence, l'acide 4-[2-(5,5,8,8 tetramethyl-5,6,7,8 tetrahydronaphtalène-2-yl)propenyl]-benzoique sont réalisées en plaque 96 puits. Le produit à tester est utilisé à 10 concentrations et l'agoniste de référence à 7 concentrations. Dans chaque puits, les cellules sont en contact d'une concentration du produit à tester et d'une concentration de l'agoniste de référence, l'acide 4-[2-(5,5,8,8 tetramethyl-5,6,7,B tetrahydronaphtalène-2-yl)propenyl]-benzoïque. Des mesures sont également réalisées pour les témoins agoniste total (l'acide 4-[2-(5,5,8,8-tetramethyl-5,6,7,8-tetrahydronaphtalène-2-yl)propenyl]-benzoique) et agoniste inverse, l'acide 4-{(E)-3-[4-(4-tert-Butyl-phenyl)-5,5,8,8-tetramethyl-5,6,7,8-tetrahydro-naphtalen-2-yl]-3-oxo-propenyl}-benzoïque.

Ces courbes croisées nous permettent de déterminer les AC5O (concentration à laquelle on observe 50% d'activation) du ligand de référence à différentes concentrations de produit à tester. Ces AC5O sont utilisées pour calculer la régression de Schild en traçant une droite répondant à l'équation de Schild ("quantitation in receptor pharmacology" Tervy P.Kenakin, Receptors and Channels, 2001, 7, 371-385*).*

Les lignées cellulaires HeLa utilisées sont des transfectants stables contenant les plasmides ERE-βGlob-Luc-SV-Neo (gène reporter) et RAR (α, β, γ) ER-DBD-puro. Ces cellules sont ensemencées en plaques 96 puits à raison de 10 000 cellules par puit dans 100 µl de milieu DMEM sans rouge de phénol et supplémenté par 10% de sérum de veau délipidé. Les plaques sont ensuite incubées à 37°C, 7% CO₂ pour 4 H.

Les différentes dilutions des produits à tester, du ligand de référence (l'acide 4-[2-(5,5,5,8 tetramethyl-5,6,7,8 tetrahydronaphtalene-2-yl)propenyl]-benzoïque), du témoin 100% (l'acide 4-[2-(5,5,8,8 tetramethyl-5,6,7,8 tetrahydronaphtalene-2-yl)propenyl]-benzoïque 100 nM) et du témoin 0% (l'acide 4-{(E)-3-[4-(4-tert-Buyl-phenyl)-5,5,8,8-tetramethyl-5,6,7,8-tetrahydro-naphtalen-2-yl]-3-oxo-propenyl}-benzoïque 500 nM) sont rajoutées à raison de 5 µl par puits. Les plaques sont ensuite incubées 18 heures à 37°C, 7% CO₂. Le milieu de culture est éliminé par retournement et 100 µI d'un mélange 1:1 PBS/Luciferine est ajouté à chaque puit. Après 5 minutes, les plaques sont lues par le lecteur de luminescence.

| | RARα Kdapp | RARβ Kdapp | RARγ Kdapp |
|---|---|---|---|
| | (nM) | (nM) | (nM) |
| Composé de l'exemple 1 | 625 | 185 | 37,5 |
| Composé de l'exemple 2 | 3000 | 600 | 120 |
| Composé de l'exemple 3 | 250 | 120 | 15 |
| Composé de l'exemple 4 | 1002 | 252 | 30 |
| Composé de l'exemple 5 | 4015 | 2015 | 120 |
| Composé de l'exemple 6 | 250 | 500 | 60 |
| Composé de l'exemple 7 | 120 | 15 | 2 |
| Composé de l'exemple 8 | 250 | 60 | 4 |
| Composé de l'exemple 9 | 60 | 60 | 0.5 |
| Composé de l'exemple 10 | 1000 | 120 | 1 |
| Composé de l'exemple 11 | 1000 | 60 | 30 |

Les résultats obtenus avec les composés de l'invention montrent bien des Kdapp inférieurs ou égaux à 1000 nM.

### Exemple 13 : Exemples de formulation

Dans cet exemple, on a illustré diverses formulations concrètes à base des composés selon l'invention.

### A/ Voie orale

| (a) Comprimé de 0,2 g | |
|---|---|
| Composé de l'exemple 5 | 0,001 g |
| Amidon | 0,114 g |
| Phosphate bicalcique | 0,020 g |
| Silice | 0,020 g |
| Lactose | 0,030 g |
| Talc | 0,010 g |
| Stéarate de magnésium | 0,005 g |
| | |

| (b) Suspension buvable en ampoules de 5 ml | |
|---|---|
| Composé de l'exemple 3 | 0,001 g |
| Glycérine | 0,500 g |
| Sorbitol à 70% | 0.500 g |
| Saccharinate de sodium | 0,010 g |
| Parahydroxybenzoate de méthyle | 0,040 g |
| Arome | qs |
| Eau purifiée | qsp 5 ml |
| | |

| (c) Comprimé de 0,8 g | |
|---|---|
| Composé de l'exemple 2 | 0,500 g |
| Amidon prégélatinisé | 0,100 g |
| Cellulose microcristalline | 0,115 g |
| Lactose | 0,075 g |
| Stéarate de magnésium | 0,010 g |
| | |

| (d) Suspension buvable en ampoules de 10 ml | |
|---|---|
| Composé de l'exemple 2 | 0,200 g |
| Glycérine | 1,000 g |
| Sorbitol à 70% | 1,000 g |
| Saccharinate de sodium | 0,010 g |
| Parahydroxybenzoate de méthyle | 0,080 g |
| Arome | qs |
| Eau purifiée | qsp 10 ml |

### B/ Voie parentérale

| (a) Composition | |
|---|---|
| Composé de l'exemple 3 | 0,002 g |
| Oléate d'éthyle | qs 10g |

| (b) Composition | |
|---|---|
| Composé de l'exemple 1 | 0,05% |
| Polyéthylène glycol | 20% |
| Solution de NaCl à 0.9% | qs 100 |
| | |

| (c) Composition | |
|---|---|
| Composé de l'exemple 3 | 2,5% |
| Polyéthylène glycol 400 | 20% |
| Solution de NaCl à 0.9% | qs 100 |
| | |

| (d) Composition de clyclodextrine injectable | |
|---|---|
| Composé de l'exemple 3 | 0,1 mg |
| β-cyclodextrine | 0.10 g |
| Eau pour injectable | qsp 10,00 g |

### C/ Voie topique

| (a) Onguent | |
|---|---|
| Composé de l'exemple 2 | 0,020 g |
| Myristate d'isopropyle | 81,700 g |
| Huile de vaseline fluide | 9,100 g |
| Silice ("Aérosil 200" vendue par DEGUSSA) | 9,180 g |
| | |

| (b) Onguent | |
|---|---|
| Composé de l'exemple 5 | 0,300 g |
| Vaseline blanche codex | qsp 100 g |
| | |

| (c) Crème Eau-dans-huile non ionique | |
|---|---|
| Composé de l'exemple 2 | 0,100 g |
| Mélange d'alcools de lanoline émulsifs, de | |
| cires et d'huiles ("Eucerine anhydre" vendu | |
| par BDF) | 39,900 g |
| Parahydroxybenzoate de méthyle | 0,075 g |
| Parahydroxybenzoate de propyle | 0,075 g |
| Eau déminéralisée stérile | qsp 100 g |
| | |

| (d) Lotion | |
|---|---|
| Composé de l'exemple 2 | 0,100 g |
| Polyéthylène glycol (PEG 400) | 69,900 g |
| Ethanol à 95% | 30,00 g |
| | |

| (e) Onguent hydrophobe | |
|---|---|
| Composé de l'exemple 2 | 0,300 g |
| Myristate d'isopropyle | 36,400 g |
| Huile de silicone ("Rhodorsil 47 V 300" | |
| vendu par RHONE-POULENC) | 36,400 g |
| Cire d'abeille | 13,600 g |
| Huile de silicone ("Ail 300.00 cst" vendu par GOLDSCHMIDT) | qsp 100 g |
| | |

| (f) Crème Huile-dans-Eau non ionique | |
|---|---|
| Composé de l'exemple 5 | 1,000 g |
| Alcool cétylique | 4,000 g |
| Monostéarate de glycérol | 2,500 g |
| Stéarate de PEG 50 | 2,500 g |
| Beurre de karité | 9,200 g |
| Propylène glycol | 2,000 g |
| Parahydroxybenzoate de méthyle | 0,075 g |
| Parahydroxybenzoate de propyle | 0,075 g |
| Eau déminéralisée stérile | qsp 100 g |

## Revendications

1. Composés de formule générale (I) suivante : 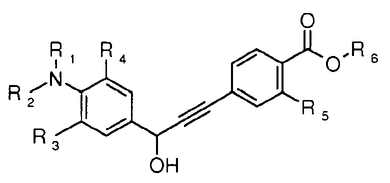 dans laquelle :
- R₁ est un atome d'hydrogène, un radical alkyle contenant de 1 à 4 atomes de carbone,
- R₂ est un radical alkyle contenant de 1 à 4 atomes de carbone,
- ou bien R₁, R₂ forment ensemble, avec l'atome d'azote N-auquel ils sont rattachés, un hétérocycle de type pipéridine ou pyrrolidine,
- R₃ est un radical alkyle contenant de 1 à 4 atomes de carbone,
- R₄ est un hydrogène, un radical alkyle contenant de 1 à 4 atomes de carbone, ou un atome d'halogène,
- R₅ est un atome d'hydrogène ou un hydroxy,
- R₆ est un atome d'hydrogène, un radical alkyle contenant de 1 à 4 atomes de carbone, ainsi que leurs sels et isomères optiques.

2. Composés selon la revendication 1, **caractérisés en ce qu'**ils présentent au moins une des caractéristiques suivantes :
- R₁ est choisi parmi un atome d'hydrogène ou un radical méthyle ou éthyle
- R₂ est choisi parmi un radical méthyle, éthyle et 2-méthylpropyle ou
- R₁, R₂ forment ensemble, avec l'atome d'azote N auquel ils sont rattachés, un hétérocycle de type pipéridine ou pyrrolidine
- R₃ est choisi parmi les radicaux 1-propyle et t-butyle
- R₄ est choisi parmi un atome d'hydrogène, un radical méthyle ou éthyle, un radical i-propyle et un atome de chlore
- R₅ est un atome d'hydrogène ou un hydroxy,
- R₆ est un atome d'hydrogène ou un radical alkyle contenant de 1 à 4 atomes de carbone.

3. Composés selon la revendication 1 ou 2, **caractérisés en ce qu'**ils présentent toutes les caractéristiques suivantes :
- R₁ est choisi parmi un atome d'hydrogène ou un radical méthyle ou éthyle
- R₂ est choisi parmi un radical méthyle, éthyle et 2-méthylpropyle ou
- R₁, R₂ forment ensemble, avec l'atome d'azote N auquel ils sont rattachés, un hétérocycle de type pipéridine ou pyrrolidine
- R₃ est choisi parmi les radicaux i-propyle et t-butyle
- R₄ est choisi parmi un atome d'hydrogène, un radical méthyle ou éthyle, un radical i-propyle et un atome de chlore
- R₅ est un atome d'hydrogène ou un hydroxy,
- R₆ est un atome d'hydrogène ou un radical alkyle contenant de 1 à 4 atomes de carbone.

4. Composés selon la revendication 1, **caractérisés en ce qu'**ils se présentent sous forme d'un sel choisi parmi les sels d'un métal alcalin ou alcalino-terreux, les sels de zinc et les sels d'une amine organique.

5. Composés selon la revendication 1, **caractérisés en ce que** R₁ est un atome d'hydrogène ou un radical alkyle contenant de 1 à 4 atomes de carbone, et R₂ est un radical alkyle contenant de 1 à 4 atomes de carbone, et **en ce qu'**ils se présentent sous forme d'un sel choisi parmi les sels d'acide inorganique et les sels d'acide organique.

6. Composé selon la revendication 1, **caractérisé en ce qu'**il est choisi parmi :
1/- Acide 4-[(*tert*-butyl-diethylamino-phenyl)-hydroxy-prop-l-ynyl]-benzoïque.
2/- Acide 4-{[*tert* - butyl -(ethyl-isobutyl-amino)-phenyl]-hydroxy-prop-1-ynyl}-benzoïque
3/- Acide 4-[3-(3- *tert -* butyl 4-dimethylamino-phenyl)-3-hydroxy-prop-1-ynyl]-benzoïque
4/- Acide 4-[3-(3- *tert -* butyl -4-pyrrolidin-1-yl-phenyl)-3-hydroxy-prop-1-ynyl]-benzoïque
5/- Acide 4-[3-(3- *tert -* butyl -4-piperidin-1-yl-phenyl)-3-hydroxy-prop-1-ynyl]-benzoïque
6/- Acide 4-[3-(3- *tert -* butyl -4-diethylamino-phenyl)-3-hydroxy-prop-1-ynyl]-2-hydroxy-bcnzoïque
7/- Acide 4-[3-(3- *tert -* butyl -5-chloro-4-dimethylamino-phenyl)-3-hydroxy-prop-1-ynyl]-benzoïque
8/- Acide 4-[3-(3- *tert -* butyl -5-chloro-4-dimethylamino-phenyl)-3-hydroxy-prop-1-ynyl]-2-hydroxy-benzoïque
9/- Acide 4-[3-(4-Dimethylamino-3,5-diisopropyl-phenyl)-3-hydroxy-prop-1-ynyl]-benzoïque
10/- Acide 4-[3-(4-Dimethylamino-3,5-diisopropyl-phenyl)-3-hydroxy-prop-1-ynyl]-2-hydroxy-benzoïque
11/- Acide 4-[3-(4-Diethylamino-3-isopropyl-phenyl)-3-hydroxy-prop-1-ynyl]-benzoïque
12/- (-) Acide 4-[3-(3- *tert -* butyl -4-diethylamino-phenyl)-3-hydroxy-prop-1-ynyl]-benzoïque
13/- (+) Acide 4-[3-(3- *tert -* butyl -4-diethylamino-phenyl)-3-hydroxy-prop-1-ynyl]-benzoïque

7. Composés selon l'une des revendications 1 à 6, à titre de médicament.

8. Utilisation d'au moins un composé selon l'une des revendications 1 à 6 pour la fabrication d'une composition destinée au traitement :
- des affections dermatologiques liées à un désordre de la kératinisation portant sur la différentiation et sur la prolifération cellulaire
- des ichtyoses, des états ichtyosiformes, de la maladie de Darrier, des kératodermies palmoplantaires, des leucoplasies et des états leucoplasiformes, du lichen cutané ou muqueux ;
- des affections dermatologiques avec une composante immuno-allergique inflammatoire, avec ou sans trouble de la prolifération cellulaire;
- de désordres cutanés dus à une exposition aux rayonnements UV, du vieillissement de la peau, photo-induit ou chronologique ou des pigmentations et les kératoses actiniques,
- des pathologies associées au vieillissement chronologique ou actinique de la peau,
- des proliférations dermiques ou épidermiques qu'elles soient bénignes ou malignes, d'origine virale ou non,
- des proliférations pouvant être induites par les ultra-violets,
- des lésions précancéreuses cutanées,
- des dermatoses immunes,
- des maladies immunes bulleuses,
- des maladies du collagène,
- des affections dermatologiques à composante immunologique ;
- des troubles ophtalmologiques,
- des stigmates de l'atrophie épidermique et/ou dermique induite par les corticostéroïdes locaux ou systémiques, ou toute autre forme d'atrophie cutanée,
- des affections d'origine virale au niveau cutané,
- des troubles de la fonction sébacée,
- des troubles de la cicatrisation, ou des vergetures, ou
- des désordres de la pigmentation.

9. Utilisation selon la revendication 8, **caractérisée en ce que** les affections dermatologiques liées à un désordre de la kératinisation portant sur la différentiation et sur la prolifération cellulaire sont choisies parmi les acnés vulgaires, comédoniennes, polymorphes, rosacées, les acnés nodulokystiques, conglobata, les acnés séniles, les acnés secondaires telles que l'acné solaire, médicamenteuse ou professionnelle.

10. Utilisation des composés selon la revendication 8 **caractérisée en ce que** les affections dermatologiques avec une composante immuno-allergique inflammatoire, avec ou sans trouble de la prolifération cellulaire sont choisies parmi le psoriasis, le rhumatisme psoriasique, l'atopie cutanée, telle que l'eczéma ou l'atopie respiratoire et l'hypertrophie gingivale.

11. Composition pharmaceutique, **caractérisée par le fait qu'**elle comprend, dans un milieu physiologiquement acceptable, au moins un composé de formule (I) selon l'une des revendications 1 à 6.

12. Composition selon la revendication 11, **caractérisée en ce que** la concentration en composés de formule (I) est comprise entre 0,001% et 10% en poids, de préférence entre 0,01 % et 1 % en poids, par rapport au poids de la composition.

13. Composition cosmétique, **caractérisée par le fait qu'**elle comprend, dans un milieu physiologiquement acceptable, au moins un composé de formule (I) selon l'une des revendications 1 à 6.

14. Composition selon la revendication 13, **caractérisée en ce que** la concentration en composés de formule (I) est comprise entre 0,001% et 3% en poids, par rapport au poids total de la composition.

15. Utilisation non thérapeutique d'une composition cosmétique selon l'une des revendications 13 ou 14 pour prévenir et/ou traiter les signes du vieillissement cutané et/ou la peau sèche.

16. Utilisation non thérapeutique d'une composition cosmétique selon l'une des revendications 13 ou 14 pour l'hygiène corporelle ou capillaire.

17. Procédé cosmétique d'embellissement de la peau, **caractérisé par le fait que** l'on applique sur la peau une composition selon l'une des revendications 13 ou 14.

## Claims

1. Compounds of general formula (I) below: 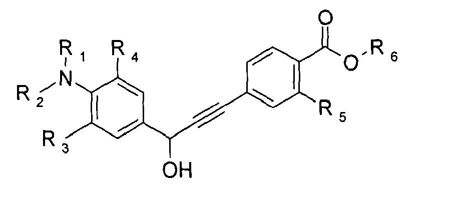 in which:
- R₁ is a hydrogen atom, or an alkyl radical containing from 1 to 4 carbon atoms,
- R₂ is an alkyl radical containing from 1 to 4 carbon atoms,
- or else R₁ and R₂ form, together with the nitrogen atom N to which they are attached, a heterocycle of piperidine or pyrrolidine type,
- R₃ is an alkyl radical containing from 1 to 4 carbon atoms,
- R₄ is a hydrogen, an alkyl radical containing from 1 to 4 carbon atoms, or a halogen atom,
- R₅ is a hydrogen atom or a hydroxyl,
- R₆ is a hydrogen atom, or an alkyl radical containing from 1 to 4 carbon atoms,
and also the salts and optical isomers thereof.

2. Compounds according to Claim 1, **characterized in that** they have at least one of the following characteristics:
- R₁ is chosen from a hydrogen atom or a methyl or ethyl radical,
- R₂ is chosen from a methyl, ethyl and a 2-methylpropyl radical or
- R₁ and R₂ form, together with the nitrogen atom N to which they are attached, a heterocycle of piperidine or pyrrolidine type,
- R₃ is chosen from i-propyl and t-butyl radicals,
- R₄ is chosen from a hydrogen atom, a methyl or ethyl radical, an i-propyl radical and a chlorine atom,
- R₅ is a hydrogen atom or a hydroxyl,
- R₆ is a hydrogen atom or an alkyl radical containing from 1 to 4 carbon atoms.

3. Compounds according to Claim 1 or 2, **characterized in that** they have all the following characteristics:
- R₁ is chosen from a hydrogen atom or a methyl or ethyl radical,
- R₂ is chosen from a methyl, ethyl and a 2-methylpropyl radical or
- R₁ and R₂ form, together with the nitrogen atom N to which they are attached, a heterocycle of piperidine or pyrrolidine type,
- R₃ is chosen from i-propyl and t-butyl radicals,
- R₄ is chosen from a hydrogen atom, a methyl or ethyl radical, an i-propyl radical and a chlorine atom,
- R₅ is a hydrogen atom or a hydroxyl,
- R₆ is a hydrogen atom or an alkyl radical containing from 1 to 4 carbon atoms.

4. Compounds according to Claim 1, **characterized in that** they are in the form of a salt chosen from the salts of an alkali metal or an alkaline-earth metal, zinc salts and the salts of an organic amine.

5. Compounds according to Claim 1, **characterized in that** R₁ is a hydrogen atom or an alkyl radical containing from 1 to 4 carbon atoms, and R₂ is an alkyl radical containing from 1 to 4 carbon atoms, and **in that** they are in the form of a salt chosen from the inorganic acid salts and the organic acid salts.

6. Compound according to Claim 1, **characterized in that** it is chosen from:
1/- 4-[(*tert*-butyldiethylaminophenyl)hydroxyprop-1-ynyl]benzoic acid
2/- 4-{[*tert-*butyl(ethylisobutylamino)phenyl]hydroxyprop-1-ynyl}benzoic acid
3/- 4-[3-(3-*tert*-butyl-4-dimethylaminophenyl)-3-hydroxyprop-1-ynyl]benzoic acid
4/- 4-[3-(3-*tert*-butyl-4-pyrrolidin-1-ylphenyl)-3-hydroxyprop-1-ynyl]benzoic acid
5/- 4-[3-(3-*tert*-butyl-4-piperidin-1-ylphenyl)-3-hydroxyprop-1-ynyl]benzoic acid
6/- 4-[3-(3-*tert*-butyl-4-diethylaminophenyl)-3-hydroxyprop-1-ynyl]-2-hydroxybenzoic acid
7/- 4-[3-(3-*tert*-butyl-5-chloro-4-dimethylaminophenyl)-3-hydroxyprop-1-ynyl]benzoic acid
8/- 4-[3-(3-*tert*-butyl-5-chloro-4-dimethylaminophenyl)-3-hydroxyprop-1-ynyl]-2-hydroxybenzoic acid
9/- 4-[3-(4-dimethylamino-3,5-diisopropylphenyl)-3-hydroxyprop-1-ynyl]benzoic acid
10/- 4-[3-(4-dimethylamino-3,5-diisopropylphenyl)-3-hydroxyprop-1-ynyl]-2-hydroxybenzoic acid
11/- 4-[3-(4-diethylamino-3-isopropylphenyl)-3-hydroxyprop-1-ynyl]benzoic acid
12/- (-)-4-[3-(3-*tert*-butyl-4-diethylaminophenyl)-3-hydroxyprop-1-ynyl]benzoic acid
13/- (+)-4-[3-(3-*tert*-butyl-4-diethylaminophenyl)-3-hydroxyprop-1-ynyl]benzoic acid.

7. Compounds according to one of Claims 1 to 6, as a medicament.

8. Use of at least one compound according to one of Claims 1 to 6, in the manufacture of a composition for use in the treatment:
- of dermatological conditions associated with a keratinization disorder relating to cell differentiation and proliferation;
- of ichthyosis, ichthyosiform conditions, Darier's disease, palmoplantar keratoderma, leukoplakia and leukoplakiform conditions, and cutaneous or mucosal lichen;
- of dermatological conditions with an inflammatory immunoallergic component, with or without a cell proliferation disorder;
- of skin disorders caused by exposure to UV radiation, photoinduced or chronological aging of the skin, or pigmentations and actinic keratosis;
- of pathological conditions associated with chronological or actinic aging of the skin;
- of dermal or epidermal proliferations, whether benign or malignant, of viral or nonviral origin;
- of proliferations that may be induced by ultraviolet radiation;
- of precancerous skin lesions;
- of immune dermatoses;
- of bullous immune diseases;
- of collagen diseases;
- of dermatological conditions with an immunological component;
- of ophthalmological disorders;
- of stigmata of epidermal and/or dermal atrophy induced by local or systemic corticosteroids, or any other form of cutaneous atrophy;
- of cutaneous conditions of viral origin;
- of sebaceous function disorders;
- of cicatrization disorders, or stretch marks; or
- of pigmentation disorders.

9. Use according to Claim 8, **characterized in that** the dermatological conditions associated with a keratinization disorder relating to cell differentiation and proliferation are chosen from common acne, comedonal acne, polymorphic acne, acne rosacea, nodulocystic acne, acne conglobata, senile acne, secondary acne such as solar acne, acne medicamentosa or occupational acne.

10. Use of the compounds according to Claim 8, **characterized in that** the dermatological conditions with an inflammatory immunoallergic component, with or without a cell proliferation disorder, are chosen from psoriasis, psoriatic arthritis, cutaneous atopy, such as eczema, or respiratory atopy and gingival hypertrophy.

11. Pharmaceutical composition, **characterized in that** it comprises, in a physiologically acceptable medium, at least one compound of formula (I) according to one of Claims 1 to 6.

12. Composition according to Claim 11, **characterized in that** the concentration of compounds of formula (I) is between 0.001% and 10% by weight, preferably between 0.01% and 1% by weight, relative to the weight of the composition.

13. Cosmetic composition, **characterized in that** it comprises, in a physiologically acceptable medium, at least one compound of formula (I) according to one of Claims 1 to 6.

14. Composition according to Claim 13, **characterized in that** the concentration of compounds of formula (I) is between 0.001% and 3% by weight, relative to the total weight of the composition.

15. Nontherapeutic use of a cosmetic composition according to either of Claims 13 and 14, for preventing and/or treating the signs of skin aging and/or dry skin.

16. Nontherapeutic use of a cosmetic composition according to either of Claims 13 and 14, for body or hair hygiene.

17. Cosmetic method for enhancing the appearance of the skin, **characterized in that** a composition according to either of Claims 13 and 14 is applied to the skin.

## Patentansprüche

1. Verbindungen der folgenden allgemeinen Formel (I): 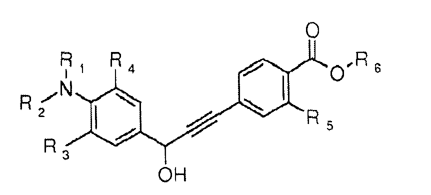 in der Formel:
• R₁ ist ein Wasserstoffatom, eine Alkylgruppe mit 1 bis 4 Kohlenstoffatomen,
• R₂ bedeutet eine Alkylgruppe mit 1 bis 4 Kohlenstoffatomen,
• oder R₁ und R₂ bilden gemeinsam mit dem Stickstoffatom N, an das sie gebunden sind, einen Heterocyclus vom Typ Piperidin oder Pyrrolidin,
• R₃ bedeutet eine Alkylgruppe mit 1 bis 4 Kohlenstoffatomen,
• R₄ bedeutet ein Wasserstoffatom, eine Alkylgruppe mit 1 bis 4 Kohlenstoffatomen oder ein Halogenatom,
• R₅ ist ein Wasserstoffatom oder eine Hydroxygruppe,
• R₆ ist ein Wasserstoffatom, eine Alkylgruppe mit 1 bis 4 Kohlenstoffatomen,
sowie ihre Salze und optischen Isomere.

2. Verbindungen nach Anspruch 1, **dadurch gekennzeichnet, dass** sie mindestens eines der folgenden Merkmale aufweisen:
• R₁ ist ein Wasserstoffatom oder eine Methyl- oder Ethylgruppe,
• R₂ ist unter Methyl, Ethyl und 2-Methylpropyl ausgewählt,
• oder R₁ und R₂ bilden gemeinsam mit dem Stickstoffatom N, an das sie gebunden sind, einen Heterocyclus vom Typ Piperidin oder Pyrrolidin,
• R₃ ist unter Isopropyl und tert-Propyl ausgewählt,
• R₄ ist unter einem Wasserstoffatom, einer Methyl- oder Ethylgruppe, einer Isopropylgruppe und einem Chloratom ausgewählt,
• R₅ ist ein Wasserstoffatom oder eine Hydroxygruppe,
• R₆ ist ein Wasserstoffatom oder eine Alkylgruppe mit 1 bis 4 Kohlenstoffatomen.

3. Verbindungen nach Anspruch 1 oder 2, **dadurch gekennzeichnet, dass** sie alle der folgenden Merkmale aufweisen:
• R₁ ist unter einem Wasserstoffatom oder einer Methyl- oder Ethylgruppe ausgewählt,
• R₂ ist unter Methyl, Ethyl und 2-Methylpropyl ausgewählt,
• oder R₁ und R₂ bilden gemeinsam mit dem Stickstoffatom N, an das sie gebunden sind, einen Heterocyclus vom Typ Piperidin oder Pyrrolidin,
• R₃ ist unter Isopropyl und tert-Propyl ausgewählt,
• R₄ ist unter einem Wasserstoffatom, einer Methyl- oder Ethylgruppe, einer Isopropylgruppe und einem Chloratom ausgewählt,
• R₅ ist ein Wasserstoffatom oder eine Hydroxygruppe,
• R₆ ist ein Wasserstoffatom oder eine Alkylgruppe mit 1 bis 4 Kohlenstoffatomen.

4. Verbindungen nach Anspruch 1, **dadurch gekennzeichnet, dass** sie in der Form eines Salzes vorliegen, das unter den Salzen eines Alkali- oder Erdalkalimetalls, den Zinksalzen und den Salzen eines organischen Amins ausgewählt ist.

5. Verbindungen nach Anspruch 1, **dadurch gekennzeichnet, dass** R₁ ein Wasserstoffatom oder eine Alkylgruppe mit 1 bis 4 Kohlenstoffatomen bedeutet, R₂ eine Alkylgruppe mit 1 bis 4 Kohlenstoffatomen bedeutet und **dadurch**, dass sie in der Form eines Salzes vorliegen, das unter den Salzen einer anorganischen Säure und den Salzen einer organischen Säure ausgewählt ist.

6. Verbindung nach Anspruch 1, **dadurch gekennzeichnet, dass** sie ausgewählt ist unter:
1/- 4-[(tert-Butyl-diethylamino-phenyl)-hydroxy-prop-1-inyl]-benzoesäure.
2/- 4-{[tert-Butyl-(ethyl-isobutyl-amino)-phenyl]-hydroxy-prop-1-inyl}-benzoesäure
3/- 4-[3-(3-tert-Butyl 4-dimethylamino-phenyl)-3-hydroxy-prop-1-inyl]-benzoesäure
4/- 4-[3-(3-tert-Butyl-4-pyrrolidin-1-yl-phenyl)-3-hydroxy-prop-1-inyl]-benzoesäure
5/- 4-[3-(3-tert-Butyl-4-piperidin-1-yl-phenyl)-3-hydroxy-prop-1-inyl]-benzoesäure
6/- 4-[3-(3-tert-Butyl-4-diethylamino-phenyl)-3-hydroxy-prop-1-inyl]-2-hydroxy-benzoesäure
7/- 4-[3-(3-tert-Butyl-5-chlor-4-dimethylamino-phenyl)-3-hydroxyprop-1-inyl]-benzoesäure
8/- 4-[3-(3-tert-Butyl-5-chlor-4-dimethylamino-phenyl)-3-hydroxy-prop-1-inyl]-2-hydroxy-benzoesäure
9/- 4-[3-(4-Dimethylamino-3,5-diisopropyl-phenyl)-3-hydroxy-prop-1-inyl]-benzoesäure
10/- 4-[3-(4-Dimethylamino-3,5-diisopropyl-phenyl)-3-hydroxy-prop-1-inyl]-2-hydroxy-benzoesäure
11/- 4-[3-(4-Diethylamino-3-isopropyl-phenyl)-3-hydroxy-prop-1-inyl]-benzoesäure
12/- (-) 4-[3-(3-tert-Butyl-4-diethylamino-phenyl)-3-hydroxy-prop-1-inyl]-benzoesäure
13/- (+) 4-[3-(3-tert-Butyl-4-diethylamino-phenyl)-3-hydroxy-prop-1-inyl]-benzoesäure.

7. Verbindungen nach einem der Ansprüche 1 bis 6 als Arzneimittel.

8. Verwendung mindestens einer Verbindung nach einem der Ansprüche 1 bis 6 für die Herstellung einer Zusammensetzung, die für die Behandlung vorgesehen ist von:
- dermatologischen Erkrankungen, die mit einer Verhornungsstörung verbunden sind, die auf der Differenzierung und Proliferation der Zellen beruht;
- Ichthyosis, ichtyosisartigen Zuständen, der Darier Krankheit, Palmoplantarkeratosen, Leukoplakien und leucoplakieformen Zuständen und Lichen der Haut oder der Schleimhäute;
- dermatologischen Erkrankungen mit immunoallergischer entzündlicher Komponente mit oder ohne Störung der Zellproliferation;
- Hauterkrankungen, die durch eine Exposition gegenüber UV-Strahlung verursacht werden, wie lichtinduzierte oder altersbedingte Hautalterung oder Pigmentierungen und aktinischen Keratosen;
- Erkrankungen, die mit einer lichtinduzierten oder altersbedingten Hautalterung einhergehen;
- Proliferationen der Dermis oder Epidermis, die gutartig oder bösartig und gegebenenfalls viralen Ursprungs sein können;
- Proliferationen, die von UV-Strahlung induziert sein können;
- präkanzerösen Hautläsionen;
- Immundermatosen;
- bullösen Immunerkrankungen;
- Kollagenerkrankungen;
- dermatologischen Erkrankungen mit immunologischer Komponente;
- ophthalmologischen Erkrankungen;
- Stigmen epidermaler und/oder dermaler Atrophie, die durch lokale oder systemische Corticosteroide hervorgerufen werden, oder aller anderen Formen der Atrophie der Haut;
- Hauterkrankungen viralen Ursprungs;
- Störungen der Talgdrüsenfunktion;
- Störungen der Wundheilung oder Streifen; oder
- Pigmentierungsstörungen.

9. Verwendung nach Anspruch 8, **dadurch gekennzeichnet, dass** die dermatologischen Erkrankungen, die mit einer Keratinisierungsstörung zusammenhängen, die auf der Differenzierung und Proliferation beruht, unter Acne vulgaris, Acne comedonica, polymorpher Akne, Acne rosacea, nodulocystischer Akne, Acne conglobata, Acne senilis und sekundären Akneformen, wie Acne solaris, Acne medicamentosa oder Acne professionalis ausgewählt sind.

10. Verwendung nach Anspruch 8, **dadurch gekennzeichnet, dass** die dermatologischen Erkrankungen mit immunoallergischer entzündlicher Komponente mit oder ohne Störung der Zellproliferation unter Psoriasis, Psoriasis arthropathica, Atopie der Haut, wie Ekzemen, Atopie der Atemwege oder Hypertrophie des Zahnfleisches ausgewählt sind.

11. Pharmazeutische Zusammensetzung, **dadurch gekennzeichnet, dass** sie in einem physiologisch akzeptablen Medium mindestens eine Verbindung der Formel (I) nach einem der Ansprüche 1 bis 6 enthält.

12. Zusammensetzung nach Anspruch 11, **dadurch gekennzeichnet, dass** die Konzentration der Verbindungen der Formel (I) im Bereich von 0,001 bis 10 Gew.-% und vorzugsweise im Bereich von 0,01 bis 1 Gew.-%, bezogen auf das Gesamtgewicht der Zusammensetzung, liegt.

13. Kosmetische Zusammensetzung, **dadurch gekennzeichnet, dass** sie in einem physiologisch akzeptablen Medium mindestens eine Verbindung der Formel (I) nach einem der Ansprüche 1 bis 6 enthält.

14. Zusammensetzung nach Anspruch 13, **dadurch gekennzeichnet, dass** die Konzentration der Verbindungen der Formel (I) im Bereich von 0,001 bis 3 Gew.-%, bezogen auf das Gesamtgewicht der Zusammensetzung, liegt.

15. Nichttherapeutische Verwendung einer kosmetischen Zusammensetzung nach einem der Ansprüche 13 oder 14 für die Vorbeugung und/oder Behandlung der Anzeichen der Hautalterung und/ oder trockener Haut.

16. Nichttherapeutische Verwendung einer kosmetischen Zusammensetzung nach einem der Ansprüche 13 oder 14 für die Körper- oder Haarpflege.

17. Kosmetisches Verfahren zur Verschönerung der Haut, **dadurch gekennzeichnet, dass** auf die Haut eine Zusammensetzung nach einem der Ansprüche 13 oder 14 aufgetragen wird.
